# EUROPEAN PATENT APPLICATION

(11) **EP 2 301 567 A1**
(43) Date of publication of application: **30.03.2011**
(21) Application number: 10184206.0
(22) Date of filing: 26.02.1998
(51) Int. Cl.: A61K 39/00, A61P 37/06

(54) **Selective immune down regulation (SIDR) for transplantation**

(30) Priority: 28.02.1997 US 808629
(62) Divisional of application: 98912887.1
(71) Applicant: ENZO THERAPEUTICS, INC., New York, NY 10022 (US)
(72) Inventor: Roy-Chowdhury, Jayanta, New Rochelle, NY 10804 (US); Ilan, Yaron, Jerusalem (IL); Rabbani, Elazar, New York, NY 10003 (US); Engelhardt, Dean, New York, NY 10025 (US)
(74) Representative: Vossius & Partner

(57) **Abstract**

Novel immune modulating processes are provided in which the immunological state of a subject including mature subjects, mammals and humans, are down regulated in a selective manner, and as a subset in a dominant manner. The novel immunological state termed SIDR for selective immune down regulation is usefully applied to the immunological modulation or regulation of gene delivery components, artificially expressed genes, gene delivery systems and expression products of artificially introduced genes by such delivery systems, and infectious agents. SIDR is also useful when combined with other immune modulating treatments such as general immune suppression and anti-apoptosis. SIDR may also be used to selectively down regulate the immune response system of a subject to a wide variety of noncellular immunogenic components and to native antigens. Other processes for producing immune suppression by administering macromolecules or compounds to a subject so as to obtain or effect SIDR are also provided. Kits for carrying out the novel processes are also provided.

## Description

### FIELD OF THE INVENTION

This invention relates to the field of immunology and to novel processes for the modulation of immune responses including particularly the down regulation of the immune response system using procedures or combinations of procedures for producing and applying a new and unexpected immune modulation termed selective immune down regulation.

All patents, patent applications, patent publications, scientific articles, and the like, cited or identified in this application are hereby incorporated by reference in their entirety in order to describe more fully the state of the art to which the present invention pertains.

### BACKGROUND OF THE INVENTION

While immunological responses are essential in animals and man, certain undesirable consequences of these responses may occur and often do. A partial list of examples of such undesirable responses include autoimmune disease, serum sickness, rejection of cell, tissue and organ transplants, rejection of (desirable non-native components, immune complex-based destruction of certain tissues, tissue and cell destruction based on the induction of apoptosis or cell death, curtain actions of antiidiotypic antibodies and certain anaphylactic responses.

One aspect of this problem revolves around the unwanted immune response to the production or presentation of non-native compounds, proteins or other antigenic materials (not found in the subject in which they have been placed) or proteins or other antigenic materials which are native constituents of the subject, but for some reason have been rendered immunogenic. In the latter instance, these specific proteins or other antigenic materials could have been rendered immunogenic for a number of reasons. They may have been modified to express a new antigenic determinant. An aberrant site of expression may have developed or even the amount of these proteins may have been altered rendering them immunogenic.

Unwanted host immune response can occur in the course of gene therapy. The immune response can be directed against antigens present in the vector and/or the products of the transferred genes. In general, the undesired production or presentation of antigens can result from the use of any viral or non-viral gene delivery system. Such an immune response can shorten the duration of expression of transgene and can substantially reduce or inhibit a repeat of the transduction to reinstate these genes, thus posing a major hurdle to long-term gene therapy.

While dependence and reliance on immune response is both necessary and required, strategies to prevent or to overcome the undesirable consequences of certain immunological responses, are limited, and often ineffective. In general, immunological suppressive methods and procedures lead to overall suppression of the immune system. Maintaining a prolonged state of immunological suppression by overall suppression of the immune system is not desirable if it can be avoided.

In contrast to general immunosuppression, tolerance to specific antigens (such as adenovirus particles) can be induced if the antigen is injected into a neonate or into a fetus (Takahashi et al.. J Biol Chem:271;26536-26534 (1996)): (Hugstrom et al. Proc Natl Acad Sci 93-3056-01 (1996)), However, this procedure has a major limitation. lt is effective only in the fetus or during the first few days after birth (not an adult).

Another tolerization protocol involves the direct injection of a soluble antigen, into the functional thymus. Man, et al., J Clin Invest 98:2640-2647 (1996). This modality of tolerization is not applicable to adult subjects since such subjects lack an active thymus (not an adult).

Certain infections could lead to an autoimmune response in which both infected and/or uninfected cells are subjected to an undesirable immune response. Examples of such responses are hepatitis B infection. HIV infection and rheumatic fever. Because immune response complications are intermingled with the element of infection, there is currently no effective cure or management strategy for these diseases.

Furthermore, the use of many non-native compounds (adenovirus, for example that are immunogenic in a subject, is also limited or inhibited due to the immunological response of the subject to these compound and reagents.

Immunological modulation is an artificially induced variation in a subject's immune system in response to the introduction of reagents, procedures and processes. Such modulation could be based on an immune response that is humoral or cellular or both which in turn occurs in response to a non-native compounds, Immunological modulation could be used to suppress an immunological response broadly or narrowly.

### SUMMARY OF THE INVENTION

Novel processes and kits for producing selective immune down regulation (SIDR) and immune suppression in subjects are provided by this invention. Among the novel processes are those for producing SIDR in an adult subject to gene delivery components. SIDR is produced in such a subject by introducing a reagent or a combination of reagents capable of producing SIDR.

Other novel processes and kits for producing SIDR includes those in which an adult subject is challenged by an artificially expressed gene. In such other processes, the SIDR is produced by introducing into the subject a reagent or combination of reagents capable of producing SIDR in which a product or a product fragment artificially expressed from the gene in question is formulated into such a reagent or combination of reagents.

This invention additionally provides novel processes for producing SIDR in an adult subject that is directed to both a gene delivery system and to an expression product from an artificially introduced gene by such delivery system. A reagent or combination of reagents capable of producing SIDR are introduced into the adult subject, the reagent or combination comprising a component or components from the gene delivery system and a product or product fragment expressed from the artificially introduced gene. A kit useful for carrying out such novel processes is also provided by this invention,

Another unique aspect of this invention concerns processes for producing SIDR in any subject to a wide variety of infectious agents, including bacteria viruses and fungi, in this aspect, a reagent or combination of reagents are introduced into the subject wherein the reagent or combination of reagents are capable of producing SIDR and they comprise some part of the infectious agent in question, be it a component or components or a fragment or fragments. A kit is also provided in which the SIDR producing reagent or reagent combination is formulated as an element for carrying out this process.

Another important feature of this invention relates to processes and kits for producing immunological tolerance in any subject. e.g., a mammal such as human. In this feature, the subject is treated, exposed or subjected to more than one immune modulating treatments or regimen - at least one of which must be SIDR. The other treatment can also be SIDR, or it can take the form of general immune suppression or anti-apoptosis.

This invention is also related to novel processes for producing SIDR in any subject to a widely diverse range of noncellular components capable of biological function or interfering with biological function in any subject. In these processes, a reagent or a combination of reagents having SIDR capability are introduced into a subject. The noncellular components are numerous and diverse covering such things as antibodies, antibody/antigen complexes, antibody/antigen cell matrices, enzymes, antitumor proteins, protein inhibitors, receptors, hormones, ligands, effectors; inducers and combination of the like. Reagents or combinations of reagents can be usefully formulated into kits for carrying out such novel processes as just briefly described.

Another feature of this invention relates to processes for producing SIDR in any subject to a native antigen or a group of native antigens. To so produce SIDR, a subject is given or exposed, treated or subjected to two or more separate and distinct immune modulating treatments, one of which must be oral tolerization as described in further detail below.

Other novel processes are provided in this invention. One such process concerns immune suppression production in a subject by administering macromolecules or compounds to the subject. The macromolecules or compounds are immunogenic themselves, or they possess the capability of providing immune suppression to the subject. In this novel process, the subject is treated to obtain a SIDR state to the macromolecules or compounds. By so doing, repeated use of these macromolecules or compounds can be undertaken with substantially little or greatly reduced immune response. In some instances, the immune response may be for all intents and purposes shut down with respect to the macromolecules or compounds.

Another novel process is provided where a transient SIDR state is obtained in a subject by transferring non-native cells from a donor having dominant selective immune down regulation to the subject under study,

In a further aspect of this invention processes are provided for producing SIDR in any subject to any antigen or group of antigens, including native antigens and those other antigens that have been transplanted from a donor to the subject under study, In this instance, non-native compounds or non-native immunological reagents capable of producing immune suppression are introduced into the subject. Either prior to or during the introduction, or even from before and up to and including the introduction the subject - who has been subjected to SIDR - is exposed or challenged by the antigen or group of antigens.

Also provided in this invention is a transplantation process comprising the steps of establishing selective immune down regulation in a recipient subject to the antigens of a donor, such antigens being any of the members selected from the group consisting of MHC Class l, MHC Class II and histocompatibility factors, or combinations thereof, and then introducing into the recipient subject any of the cells, tissue, organs or components thereof from the donor.

This invention also provides a transplantation process comprising the steps of establishing selective immune down regulation (SIDR) in a donor to an antigen or antigens of a recipient, the antigen or antigens of the recipient, SIDR establishment being carried out by introducing to the donor any of the cells, tissue, organs, or components thereof from the recipient. In this way, selective immune down regulation in the donor to the recipient antigen or antigens is established prior to transplanting into the donor any of the cells, tissue, organs, or components thereof. Thereafter, any of the cells, tissue, organs, or components thereof from the donor having selective down immune regulation can be transplanted into the recipient.

This invention further provides a process for treating Crohn's disease in a subject. To do so, selective immune down regulation in the subject to inflamed intestine is established by introducing into the subject components, cells, tissues or organs derived from self, rtan-self or immunologically equivalent.

Another provision of this invention is a method of treating diabetes in a subject by establishing in the subject selective immune down regulation to non-insulin epitopes. The non-insulin epitopes comprising any of the members selected from the group consisting of pancreatic cells, insulin receptors, fatty cells and liver cells, or component thereof. Such epitopes are derived from self, non-self or immunologically equivalent.

More particular details and embodiments of the invention are described more fully below in the detailed description and preferred embodiment sections of this application that follow.

### BRIEF DESCRIPTION OF THE FIGURED

**FIGURE 1** demonstrates β-galactosidase expression in liver specimens from orally tolerized rats (group B) and the control rats (Group C2) after the second injection of the recombinant virus.
**FIGURE 2** shows PCR gel results from the detection of the presence of human BUG; DNA in rat livers after the second injection of the recombinant virus.
**FIGURE 3** is a Western blot analysis of expression of human BUGT after the second injection of the recombinant virus.
**FIGURE 4** shows the results of the effect of tolerization upon bilirubin levels after the second injection of the recombinant virus.
**FIGURE 5** depicts the anti-adenovirus antibody levels in group A tolerized (solid bars) and group C control (open bars) rats-after the first and second injections of the recombinant virus.
**FIGURE 6** are micrographs of liver biopsies taken taken 24.72 hours after the second injection showing minimal lymphocytic infiltration in tolerized rats (A) and severe inflammation in the control rats (B)3.
**FIGURE 7** are PCR gel results from the detection of the presence of human BUGT, DNA in rat livers after the second injection of the recombinant virus.
**FIGURE 8** is a Western blot analysis of expression of human BUGT_{,} after the second injection of the recombinant virus.
**FIGURE 9** is a graph showing tite effect of tolerization upon bilirubin levels after the second injection of the recombinant virus.
**FIGURE 10** is a graph showing serum bilirubin levels after adoptive transfer.
**FIGURE 11** is a color micrograph for β-galactosidase expression in liver specimens from rabbits after first injection.
**FIGURE 12** is also a color micrograph for β-galactosidase expression in liver specimens from rabbits three weeks after first injection.
**FIGURE 13** is also a color micrograph for β-galactosidase expression in liver specimens from orally tolerized rabbits after second injection,
**FIGURE 14** is also a color micrograph of β-galactosidase expression in liver specimens from non-tolerized control rabbits after second injection.

### DETAILED DESCRIPTION OF THE INVENTION

The present invention provides, among other things, a new immune modulating process in which the immune response system of a subject can be specifically down regulated. This novel approach to immune modulation in which undesirable or deleterious immune reactions are specifically suppressed in a subject has been termed selective immune down regulation ar SIDR.

The present invention provides processes, kits and compositions in the form of reagents for producing this unique SIDR condition. In attaining SIDR, the present invention relies on immunomodulation procedures to facilitate the introduction or incorporation of novel biologically functional non-native compounds or non-native cellular material in a subject who can be a mammal, including a human, or an adult human. Another aspect of this invention is to uncouple the immunological response to infectious agents from the propagative aspects of said infectious agents through immunological modulation. It is a further aspect and principle of this invention that a single immunological suppression approach in and of itself cannot lead to effective inhibition of the subject's immunological system. It has now been found however, that a combination of independent and separate immunosuppression approaches in such a subject could approach or attain an effective inhibition of the immune system, in such an inhibited state, and in the presence of an immunologic stimulus (for example the introduction of an antigen or a group of antigens), the immune system of the subject will no longer remain naive with respect to said antigen or antigens, but will proceed to launch both an immune response, as well as an immune tolerance. This immunological duality - immune responsiveness and immune tolerance - allows the subject to be competent against all antigens other than the selected antigen for which tolerance is desirable. In applying combined immunosuppressive procedures and treatments that would sufficiently inhibit the immune response while allowing the development of immune tolerance, the subject will develop SIDR.

In some instances a given immune modulation that can train the immune system in itself may not produce the desired immunological or biological effects sufficiently to produce optimal results. Thus, another aspect of this invention is to improve such a process by combining at least one oral tolerization procedure with any other immune modulation procedures which could even include two or more such immune modulating procedures or treatments. Such combinations could take the form of two independent and separate selective immune down regulation procedures both directed to a given specific antigen or antigens. For example, two such selective immune down regulation procedures could comprise oral tolerization and selective immune suppression. Such a procedure could further comprise the use of other immune modulating procedures such as immunosuppressive drugs, appropriate cytokines, adjuvants, conjugates, or combinations thereof.

As used herein, "selective immune down-regulation" (SlDR) is an immunological state in a subject or biological system in which the subject maintains tolerance (prevention or suppression of a specific immune response) to a particular antigen or set of antigens (or other immunological determinant(s)) while at the same time maintaining immunological competence against other antigens, or other classes of antigens or immunological determinants. Furthermore, in such a state, SIDR is capable of being maintained in the subject after immunological processes or modulation that has led to the SIDR state has ceased or terminated.

As used herein, the term "dominant" as employed with immune down regulation or DIDR refers to a particular form of SIDR. If the SIDR state can be transferred and manifested as a dominant state in the new subject, then such a state is defined as a dominant immune down-regulation (DIDR) state.

As used herein, the term "general immune suppression or suppressive" (GIS) refers to immune modulating reagents or procedures which could lead to the prevention of an immune response that is not specific to any particular antigen or set of antigens but rather is indiscriminate, non-specific and general. Such an immune suppression can be maintained in general if the reagents or procedures are themselves maintained. Such reagents or procedures can be administered transiently, repeatedly, or over prolonged duration.

Among the novel processes provided by this invention is one for producing selective immune down regulation in an adult subject to a gene delivery component. This novel process comprises introducing into the adult subject a reagents or a combination of reagents capable of producing selective immune down regulation. In a further aspect of the just-described process, the SIDR can be dominant, a term and state defined above. The gene delivery component may take a wide variety of forms, including viral, e.g., adenovirus, and nonviral, e.g., proteins, ligands, or any protein containing or proteinaceous molecule.

The reagent or combination of reagents that are capable of producing SIDR can comprise some portion or fragment of the gene delivery component introduction of the SIDR producing reagent or combination of reagents can be carried out using conventional methodology and procedures that are well known to those skilled in this art. For example, the reagent or combinations can be introduced continuously into the subject, or introduced in a series of separate administrations. The separate administrations may be marked by fixed time intervals or variable time intervals, as the case may be. For a further description of administration and protocols for introducing the reagents or combination of reagents, reference is made to Oral Tolerance: Mechanism and Applications, H.L. Weiner and L.F. Mayer, eds. (1996) The New York Acadamy of Sciences New York, N.Y., the contents of which are incorporated by reference.

In another aspect of the aforementioned process, one or more anti-apoptotic agents may be administered to the adult subject. Apoptosis refers to an evolutionarily conserved form of cell suicide and is well described. See, for example, the review article by Wyllie, et al., "Cell Death: The Significance of Apoptosis", International Review of Cytology, Vol. 68. See also, the review articles by Sachs, et al., Blood, 82: 15-21 (1993), Kerr, et al., Br.J. Cancer, 26: 239-257 (1972), and the more recent review article by Thompson, Science, 267: 1456-1462 (1995). All of the foregoing are incorporated by reference. The latter article is particularly useful because it provides several inhibitors of apoptosis on page 1457. These inhibitors or anti-apoptotic agents include a number of physiologic inhibitors, viral genes and pharmacological agents, any or all of which can be used in the instantly described process. A number of textbooks specificially dealing with apaptosis have been published. These include, for example. Tomei's Apoptosis: The Molecular Basis of Cell Death, Cold Spring Harbor Laboratory, volumes 3 (1991) and 8 (1994); Kroemer's Apoptotis In Immunology, Springer Verlag, Inc. (1995); and Gregory's Apoptosis And The Immune Response, (1995). The contents of all the foregoing review articles and textbooks are incorporated herein by reference. In a particular aspect of the process, the anti-apoptotic agent can comprise an antibody directed against an apaptotie factor or an antibody directed against a cytokine, including lymphokines,

To carry out the process and to produce SIDR in an adult subject to a gene delivery component, the present invention contemplates a kit useful for that purpose. The kit comprises in packaged combination or containers a reagent or reagents or particular combinations of reagents capable of producing SIDR to the gene delivery component. These reagents have been described above. Buffers and instructions are other conventional elements of the kit.

Still another process provided herein produces SIDR in an adult subject to an artificially expressed gene, the gene being expressed within the adult subject. In this process a reagent or combination of reagents are formulated based upon a product or product fragment expressed from the gene of interest. These reagents or the combination of reagents are capable of producing SIDR in an adult subject when so formulated. Further, the reagent or combination of reagents that are introduced into the adult subject can themselves be capable of producing dominant immune down regulation (DIDR).

In terms of the artificially expressed gene, it may be native or non native to the subject, and it will be non viral. A delivery system for such a gene may be viral (e.g., adenovirus) or non-viral. As in the case of other novel processes of this invention, one or more anti-apoptotic agents may be administered to the subject. These agents include any of those selected from physiologic inhibitors, viral genes and pharmacological agents or combinations thereof, including antibodies directed against apoptotie factors or cytokines, as described above and elsewhere. See, for example, Thompson, (1995), supra.

The present invention also provides a kit useful for producing selective immune down regulation in an adult subject to an artificially expressed gene, said kit comprising in packaged combination or containers a reagent or reagents capable of producing selective immune down regulation in an adult subject, and buffers and instructions therefor.

Still yet another novel process provided by this invention produces SIDR, a process for producing selective immune down regulation in an adult subject both to a gene delivery system or component thereof and to a product from expression of an artificially introduced gene, the gene having been introduced into the adult subject by the aforementioned gene delivery system. This process composes introducing into the adult subject a reagent or a combination of reagents capable of producing SIDR. The reagent or reagents or combination of reagents comprise a component or components of the gene delivery system and a product or product fragment expressed from the gene in question. As in the case of other novel processes described above, this process can also be dominant, or the reagents or combination of reagents may be capable of producing DIDR. The nature of the gene (for example, native or non native, viral or non viral, the gene delivery system or component (for example, viral or non viral), the subject (mammal such as human) and the further aspect of administering anti-apoptotic agents (for example, physiologic inhibitors, viral genes and pharmacological agents or antibodies, are all as described above.

In conjunction with the just described process, the present invention also provides a kit useful for producing SIDR also in an adult subject to the gene delivery or to expression of an artificially introduced gene, in the adult subject. The kit comprises, in packaged combination or containers, (i) a reagent or a combination of reagents capable of producing selective immune down regulation and, (ii) one or more anti-apoptotic agents, and buffers and instructions therefor. The latter component (ii) represents an optional element of the kit and is designed for a specific preferred aspect of the novel process herein above described.

A particularly useful application of SIDR involves infectious agents. Here, the novel process produces SIDR in any subject (for example, mammals and humans) to an infectious agent, the latter assuming any number of diverse forms and types, including bacteria, viruses and fungi. Among suitable candidates for viral infectious agents are those selected from the following group: hepatitis B virus (HBV), hepatitis C virus (HCV), human immuno deficiency virus types 1 and 2 (HIV-1 and HIV-2), human T-cell leukemia virus type 1 (HTLV-1), cytomegalovirus (CMV), Epstein-Barr virus (EBV), and herpes simplex virus (HSV). The foregoing list of viral infectious agents is in no way intended to be exhaustive and may very well include others.

The infectious agent component or components or fragments thereof can be contained within a cell matrix of the subject or they can be complexed with cell receptors or antibodies from the subject or with any conjugates derived from such component, components, fragments, complexes and the like.

As in the case of other novel processes described herein above, the SIDR in this process can be dominant or the reagent or combination of reagents can possess the capability of producing DIDR. See the above definition for DIDR. DIDR may be effected or obtained by administering to the subject at least one component or a fragment of the infectious agent or even a cell containing a component or a fragment of the infectious agent.

In accordance with a general principle and useful application of SIDR, the SIDR subject can be further treated with a variety of compounds or drugs directed against pathogens. These would include any of the anti-viral compounds, anti-bacterial compounds and anti-fungal compounds. Among the anti-viral compounds are those from the following groups: chemotherapeutic agents, enzyme inhibitors and interferons. The nature, availability and sources, and the administration of all such compounds including the effective amounts necessary to produce desirable effects in a subject are well known in the art and need not be further described herein.

As in the case of other previously described novel processes, anti-apoptotic agents can be administered to the subject as part of the SIDR producing process. In addition, at least one other immune modulating treatment, e.g., general immune suppression and SIDR, can also be employed in the process.

For carrying out the just described process against infectious agents the prevent invention also provides a kit useful for producing SIDR in a subject to an infectious agent. In this instance, the kit comprises in packaged combination or containers a reagent or a combination of reagents capable of producing SIDR, and also comprising a component or components or fragments thereof of the infectious agent in question. Buffers and instructions may also be included in this kit.

One extremely useful application of the present invention is the use of SIDR in combination with other conventional immune modulating treatments. The general immunological suppression procedure can take the form of immunosuppressive drugs, such as cyclosporin or other such drugs, or antibodies to immune cells such as anti-CD4, anti-CD8, OTK, etc. or cytokines or sub-ablative doses of radiation. Immunologic modulation leading to SIDR includes development of specific tolerance by use of general immune suppressors such as CD4 or CD8 antibodies or a combination with other anti-lymphocyte antibodies. While exposing the subject to continuous presence of specific antigenic Immunosuppressive compounds and drugs that are well described in the literature. See for example. Benjamini's Immunology: A Short Course, 2nd Edition, Wiley-Liszt Inc. (1991), Chapter 19 "Transplantation immunology," pp. 347-367; and Stites' Basic and Clinical Immunology, 7th Edition, Appleton & Lange, Norwalk, CT (1991). Chapter 61 "Immunosuppressive Therapy," pp. 766-779. For more recent textbooks on this subject, please refer to Rich's Clinical Immunology: Principles and Practice, Mosby, St. Louis, MO, and Samter's lmmunologic Diseases, 5th Edition, Little, Brown and Company, Boston. All of the foregoing are incorporated by reference.

As an aspect of the combination treatment approach, the present invention also provides a process for producing immunological tolerance in a subject to a gene delivery component or to an artificially expressed gene in the subject, or to both the gene delivery component and to a product expressed from the gene. Here in this novel process, the subject is exposed, treated or otherwise subjected to more than one immune modulating treatment, at least one of which treatment is SIDR. The other treatment or treatments are selected from general immune suppression, anti-apoptosis and SIDR. Thus, in another aspect, two immune modulating treatments can be deployed for purposes of the just described process. For example, SIDR and GIS. SIDR and anti-apoptosis and SIDR, GIS and anti-apoptosis can all be usefully combined as such to produce immunological tolerance in the subject.

The two or more immune modulating treatments can be administered prior tn administration of the gene delivery component or the expression of the artificially expressed gene, or both. Alternatively, the two or more immune modulating treatments can be administered after the gene delivery component or expression of the artificially expressed gene. Alternatively, the two or more treatments can be administered at substantially or approximately the same time as the gene delivery component or the expression of the artificially expressed gene. In another aspect, the subject can be exposed simultaneously to the two or more immune modulating treatments or the subject may be exposed to the treatments at different times. The administration including specific reagents or drugs concentrations, mode of administration, monitoring, duration of administration and the like are routinely encountered in the clinical setting and would represent, therefore, information known or available to those skilled in the art,

As in the case of other novel processes herein before described, the nature of SIDR (for example, dominant or DIDR), the gene delivery component (viral or non viral), the expressed gene and product or fragment expressed therefrom (viral or non viral), the immunosuppressive compounds (corticosteroid), cytotoxic drugs, cyclosporine and anti-lymphocyte antibodies (polyclonal or monoclonal), and anti-apoptosis treatment (physiologic inhibitors, viral genes and pharmacological agents, and the subject (mammals and humans) can take any of the forms previously described herein. It should also be noted that in the aforedescribed process that both the gene delivery component can be introduced into the subject and the gene can be artificially expressed as well.

In conjunction with this process, the present invention also provides a kit for producing SIDR in a subject to a gene delivery component or to an artificially expressed gene. In packaged combination or containers, the kit comprises a reagent or a combination of reagents capable of producing SIDR, and at least one other means for generating general immune suppression, or anti-apoptotic effects in the subject, or both. Buffers and instructions can also be included in the kit.

The present invention is also applicable to processes for inducing SIDR in donor (management of the donor in transplantation) against the recipient cells in order to prevent rejection of the recipient cells by the donor cells, for example, in a bone marrow transplant system. Additionally, the specific suppression of undesirable immune reactions in adults can be attained or approached using the present invention. As described earlier, this may be achieved by a variety of means either alone or in combination. In a preferred mode, SIDR can he used to suppress immune reactivity to antigens carried by recombinant viral vectors. The tolerization can be carried out before or during the course of expression of the viral vector, or it can be carried out after an immune reaction to one or more of the viral antigen has already been established.

The immunological tolerance may be induced (tolerization) by injecting viral antigens directly into the spleen or into the hepatic portal vein of the target animal (Cantor, et al., Nature 215:744-46, 1967)

The immunological determinants that are subject to the methods and compositions of immodulation of the present invention are comprised of one or more antigens. These antigens can be native or non-native with regard to the subject. They can be natural or synthetic, modified or unmodified, whole or fragments thereof. Fragments can be derived from synthesis as fragments or by digestion or other means of modification to create fragments from larger entities. Such antigen or antigens comprise but are not limited to proteins, glycoproteins, enzymes, antibodies, histocompatibility determinants, ligands, receptors, hormones, cytokines, cell membranes, cell components, viruses, viral components, viral vectors, non-viral vectors, whole cells, tissues or organs. The antigen can consist of single molecules or mixtures of diverse individual molecules. The antigen can present itself within the context of viral surface, cellular surface, membrane, matrix, or complexed or conjugated with a receptor, ligand, antibody or any other binding partner. Such antigen or antigen can be introduced to the subject alone or with agent or agents that could further contribute delivery, uptake, stability, reactivity or targetting.

The antigen in some applications of the present invention will be introduced into the subject for two independent objectives. In the first instance, the antigen is introduced into the subject by means of an appropriate protocol so as to produce a state of selective immune down regulation (SIDR) in said subject. In the second instance the antigen (a non native compound) is introduced into the said subject so as to provide biological function in such subject. It is further understood that there may be only immunological equivalency between the antigen or antigens used to produce SIDR and the non-native compound with biological function i.e. structurally they do not have to be identical. It is further understood that state of SIDR can be obtained in a subject wherin the subject is not only tolerant to the immunological determinant used to create the state of SIDR but it may be further tolerant to other compounds that contain the immunological determinant.

The production of SIDR in subject to noncellular immunogenic components is also an important aspect of this invention. Thus, there is provided a process for producing SIDR in any subject to a nonviable immunogenic component, which component is capable of biological function itself or is capable of interfering with a biological function in the subject. In this process, a reagent or a combination of reagents capable of producing SIDR are introduced into the subject. SIDR may be dominant as described earlier. The noncellular immunogenic component can take a number of diverse forms, including but not limited to an antibody, an antibody/antigen complex, an antibody/antigen cell matrix, an enzyme, an antitumor protein or protein inhibitor, a receptor, a hormone, a ligand, an effector and an inducer, or combinations of any of the foregoing, In the case of the antibody or antibody/antigen complex, these can be polyclonal or monoclonal in nature. Furthermore, the antibody can be to one or more epitopes on an immune cell. Merely by way of example, such epitopes can include CD2, CD4, CD8, CTLA4lg. OTK, anti-Th, or combinations thereof. See Thomson's Molecular Biology of lmmunosuppression. John Wiley & Sons, Inc. (1992), for discussion of molecules and epitopes involved in immune responses. See also Tilney's Transplantation Biology: Cellular and Molecular Aspects (1996) and Kuby's immunology, Freeman, San Francisco, (1996), Chapter 24, pp. 571-573. Both textbooks are incorporated by reference. Even further, the antibody can be directed to a number of proteins or factors including, for example, an apoptotic factor, a lymphokine, a cytokinin, and a histocompatibility factor (MHC Class I and/or MHC Class II), or any such combination. Where an enzyme is contemplated, the non cellular immunogenic component can comprise a metabolic enzyme involved in the conversion, consumption or degradation of a metabolic product or intermediate. Such metabolic enzymes are well described and representative members include L-asparaginase, superoxide dismutase, bilirubin oxidase, and adenosine deaminase or combinations thereof. See, for example, Maeda, et al., Bioconjugate Chemistry, 3:128-139 (1992) for a description of the foregoing enzymes as well as other metabolic enzymes and conjugates. That article is incorporated by reference.

Also contemplated by this invention is a kit for carrying out the SIDA process involving noncellular immunogenic components. The kit comprises in packaged combination or containers a reagent or combinations of reagents capable of producing SIDR relative to the noncellular immunogenic component capable of illiciting a biological function.

These SIDR procedures are extremely advantageous over previous procedures for inducing immune suppression in that the instant processes are specific for certain antigens. Systemic administration of small molecule immune suppressors (such as cyclophosphamide) and soluble factors such as transforming growth factor beta (TGFβ) or interleukin 12 (1L12) or Interleukin 4 (1L4) and others (Lederr al., Samters Immunologic Diseases 5th ed. Little Brown, (1995). p:129-143) will suppress certain aspects of the immune response, however, they lack antigen-specificity that the strategies listed above have. (Takahashi (1992), llan (1996), supra).

Another useful application of the present invention has utility relates to gene therapy. In general the use of adenovirus as a transducing virus is limited because the presence of the transducing adenovirus in the target organism leads to a cellular and humors) immune response. Host immune response, directed at vector antigenic determinants or a transgene product, can be a potential problem limiting the use of any viral or non-viral vectors for transferring genes into living organism. These transgene can include but are not limited to antibiotic-resistance genes, any selectable markers or genes that express immunologically active products. Viruses include but are not limited to HSV, HIV-based systems, retrovirus-based transducing viruses, MMLV-based systems, SV40, polyoma. HBV, EBV, VSV, Sindbis and Semliki Forest Virus, picornaviruses and other viruses that are used for transduction in animals,

The present invention also extends to non-viral gene delivery systems since these systems may also raise an immune response. These non-viral delivery systems can include but are not limited to liposomes, the various cationic and anionic lipid delivery systems, systems that induce receptor-mediated endocytosis and systems that promote the uptake of cells of nucleic acids based on the DNA or RNA transport system. In the present invention, the administration into the patient of complexes containing the antigenic carrier (consisting of fusogenic peptide, cationic lipids, anionic lipids, histones, albumin, polylysine, polysaccharides or other components) as a tolerizing agent by SIDR and/or GIS protocols or other suitable methods, will find utility in long-term gene therapy by making repeated administrations possible. In general the present invention can be used to prepare the human or animal recipient for any gene delivery that may induce an immune response.

If a patient or an animal has preexisting antibodies and Cytotoxic lymphocytes directed against a specific antigen that is desired to be delivered to them, the present invention can be used to lower the titer of the antibody response, the T-cell mediated immune response or any effect of the immune response, (including apoptosis, anti-idiotypic response or whatever) before the antigen is added as an adjuvant. This procedure will increase the dwell time of the reagent when it is presented subsequent to the initial presentation and also improve the ability of the complex to reach the target cell.

For example, many people have experienced an adenovirus infection sometime during their lives, and may possess a high titer of anti-adenovirus antibody in circulation. Injection of recombinant adenovirus vectors into these people may not transfer effective amounts of genetic material into these subjects because the virus will be neutralized before it reaches the target cells. Futhermore those viruses that do reach the cells will induce an immune cell response that will lead to the elimination of the transduced target cell by normal Immune surveillance mechanisms or clearance of the viral vector by the effect of the cytotoxic lymphocytes. In one of the examples that follow, it is shown that when an animal is first immunized against adenovirus such that a high titer of antibodies is induced, a subsequent SIDR protocols reduces the antibody titer to the point that recombinant adenovirus injected intranvenously can be expressed in the hepatocytes of the tolerized animal. The oral tolerizing protocol reduces the preexisting antibody levels and eliminate the synthesis of new antibodies. In time, the circulating levels of the anti-adenoviral antibodies are eliminated.

SIDR has the effect of lengthening the time of transient expression of transducing and transfection nucleic acid delivery systems. It has previously been shown that if one injects transducing adenovirus into SCID's mice, one observes transducing gene expression for 4 or 5 months (Dai, et al.,Proc Natl Acad Sci USA 1995:92:1401-1405, incorporated by reference. This is a measure of the length of expression one expects from adenovirus transducing vectors is animals where the immune responsiveness is severly limited. In contrast, after injection into an immunologically functional animals which has not had previous exposure 10 adenovirus with an adenovirus-based transducing virus, expression of the transducing gene lasts only about two months (Dai (1995) supra). Thus, the immune response obviously shortens the period of transient expression of transducing genes of the virus.

T immunoregulatory cells (Ts cells) are induced either by certain antigen determinants or by the presence of specific allotypic or idiotypic determinants. Once these cells are induced, they act as memory cells capable of being reactivated throughout the lifetime of the host organism or the adaptive organism (Han, et al., Hepatology.24:304.A 1996.

It should not in any way be overlooked that SIDA can be effected or obtained by means of oral tolerization. Even more significant is the principle or observation that SIDR or DIDR can also be effected through a selective immune suppressive. Such a selective immune suppressive (SIS) can comprise any of the following immune suppressors; an antibody to a T cell, an immune suppressive drug, and a cytokine or any combination thereof. The antibody to the T-cell can he representatively selected from the following: anti-CD4, anti-CD8 and OTK, or combinations thereof. Those skilled in the art will certainly appreciate that the foregoing short list of selective immune suppressive and T-cell antibodies are exemplary and by no means exhaustive.

In yet another aspect, this invention provides a process for producing selective immune down regulation in a subject to a native antigen or group of native antigens (e.g., autoimmune antigens). In this case, the process comprises subjecting said subject to at least two separate immune modulating treatments at least one of which comprises oral tolerization. As part of this process, the native antigen or group of native antigens are derived from the subject's cell or tissue, or fragments thereof, or from the subject's cell or tissue or fragments complexed with antibodies, or from partial digests of any of the foregoing. Among representative antigens or group of antigens are those selected from collagen, islet cell, liver cell, kidney cell, heart cell, pancreatic cells, spleen cell, and nucleic acid, or combinations of the foregoing. The antigens or group of antigens can also comprise a cell or tissue (for example, bone marrow) organ or components or fragments thereof, Such things can be derived or taken from the donor's skin. The second treatment in the just described process can he selected to form SIDR, GlS or anti-apoptosis. SIDR can even be used for both or all of the separate immune modulating treatments.

In the last described process, one or more cytokines can be administered to the subject, or treatments may be administered as described earlier, for example, the separate immune modulating treatments can be given repeatedly in a single dosage or single dosage period or they can be given in separate dosage periods, In addition, the treatments can be given separately or concurrently with each other. Or they can be given with partial overlap in the dosage period. It should be noted that in implementing this process and applying it to a particular case, the subject (including mammals and human) can be sensitive or naive to the antigen or group of antigens in question. Another process unique to this invention produces immune suppression in any subject, In this instance, the process comprises administering macromolecules or compounds to the subject, the macromolecules or compounds being immunogenic or being capably of providing immune suppression, wherein the subject was previously treated to obtain SlDR to said macromolecules or compounds. This permits repeated use of the macromolecules or compounds with substantially little or no immune response.

The present invention is particularly advantageous in the field of transplantation. For example, there is provided a process for transiently producing SIDR in a subject to a specific antigen. In this process, non-native cells are transferred from a donor to the subject, wherein the donor has dominant or DIDR. For purposes of this process, the subject can tie immunosuppressed prior to or during the transferring step, or even prior to and during the transferring step.

In still yet another feature, the present invention provides a process for producing SIDR in a subject to an antigen or group of antigens. Here, non-native compounds or non-native immunological reagents capable of producing immune suppression when introduced into the subject. Prior to or during or prior to and during the introducing step, the subject is exposed to the antigen or group of antigens in question, the subject having been subjected in SIDR to the non-native compounds or non-native immunological reagents. The antigen or group of antigens can be native to the subject or they can be transplanted from a donor to the subject. The SIDR can comprise antibodies to T-cells such as those described hereinabove, including CD4, CD8 and OTK, or combinations thereof. Other drugs or biological effectors can be administered in conjunction with this process, including one or more cytokines.

Useful and serving as another important aspect of the invention related to transplantation are other novel processes. One such transplantation process comprises introducing into a recipient subject (i) a donor liver or cells from a donor liver, and (ii) cells, tissue or organs from the donor, wherein the transplanted donor liver or donor liver cells inhibit rejection of the donor cell, tissue or organ by the recipient. The cells from the donor liver can comprise immune cells or dendritic cells, Exemplary as donor cells, tissues or organs are members selected from kidney, heart, lung, pancreas, islet cells, skin, bone or cells or tissues derived from any of the foregoing,

A further transplantation process is also provided by this invention. In this process, SIDR is established to the antigens of the donor in a recipient. The cells, tissue or organs or components thereof from the donor are then introduced into the recipient subject. This process can be supplemented with other immune modulating treatments that can be administered to either the recipient subject, the donor, or both.

A further transplantation process of this invention comprises transplanting cells, tissue or organs from a donor to a recipient subject. In this process, the recipient subject has been subjected to at least two independent immune modulating treatments, at least one of which comprises selective immune down regulation. Such cells, tissue or organs from the donor can comprise bone marrow.

Transplantation of organs (e.g. kidney, liver, heart, lungs, intestines, pancreas, skin etc.) or isolated cells or cell clusters (e.g. liver cells, pancreatic islets, etc.) or tissues derived from allogeneic living or cadaver donors require prolonged generalized immunosuppression with drugs such as cyclosporine, tacrolimus (FK506), corticosteroids (e.g. prednisolone, prednisone, methyl prednisolone), azothiaprine, cyclophosphamide, certain cytotoxic reagents such as antilymphocytic globulins (ALG) or antilymphocyte monoclonal antibodies (OKT3), etc., usually in combinations. Transplantation of organs or cells derived from other species (xenografting) is also being contemplated. Currently, systemic immunosupression is used for these procedures as well, although with limited success. Prolonged generalized immunosupression leaves the subject susceptible to infections by a wide variety of organisms, including bacteria, mycoplasma, and fungi and by viruses. These subjects are also at a much higher risk of developing malignant tumors, such as lymphomas. A strategy to decrease or eliminate the need for prolonged exposure to general immune suppresser elements will be to use a combination of a process that induces specific immune down regulation of the immune response to the immunogenic elements in the donor cells, tissues or organs and one or more of the general immune suppressor elements. One example of this will be to administer, orally, specific histocompatibility antigens or other immunogenic components of the donor cells in appropriate doses and for suitable a suitable length of time to tolerize the recipient to the allograft or xenograft, These antigens can be obtained from cells of the donor (e.g. blood cells) or expressed in vitro by recombinant technology. A second combination will be the use of two or more separate processes leading to specific immune down regulation based on the transplantation of dendritic cells (ref: Clare-Salzler, M.J., Brooks, J., vanHerle, K. and Anderson, C. (1992) J. Clin invest. 90: 741-748) plus the oral administration of donor material described above. Two or more immunomodulatory agents may be used. By relieving the patient from the necessity of remaining on an immunosuppressant agent on a long term basis, this approach will avoid the risk of infection and lymphoma, and the other side effects of the immunosuppressive agents (e.g. nephrotoxicity).

Graft versus host disease (GVHD) is, a major complication of non-solid as well as some of the solid organ transplants. In bone marrow transplant recipients, GVH is a major cause of morbidity and mortality. Through the present most of the measures taken to treat this disorder involved generalized immunosuppression of the patient. Attempts to delete T cells from the donor bone marrow have been made, however, they diminished the so-called graft-versus-tumor effect (GVTE) that is important to the success of bone marrow transplantation.

In the invention, specific immune down regulation of the donor towards the recipient will be used, namely, the tolerizing of the donor against the recipient major histocompatibility, and/or other antigens will be used to render the immune cells of the donor tolerant of the recipient cellular material. The donor subject will be fed with recipient cellular material or membranes taken from various tissues most commonly involved in GVHD, such as skin, intestine and liver. Thus, by rendering the donor immunologically downregulated against the recipient, GVH can be eliminated or markedly alleviated. The graft versus tumor effect (GVTE) is not expected to be diminished as tumor cells in the recipients present different antigens than the nontumor material.

Another strategy for minimizing or eliminating transplant rejection is to use the liver as a possible tolerizing organ. It was previously shown that the liver may have a role in the induction of tolerance towards foreign antigens that are fed, or are injected into the portal vein. Specific populations of liver cells, for example, the liver dendritic cells, could selectively induce immunomodulation and/or downregulate the immune responses towards foreign antigens, including allograft and xenograft-associated antigens. A donor whole liver, or a liver lobe taken from the donor, will be transplanted alongside with another solid or non-solid organ, and will induce tolerance towards the recipients. Alternatively, cells from the donor liver will be infused into the liver of the recipient through direct injection or through injection into the portal vein or the spleen and this will induce tolerance toward the donor transplanted material. This procedure will be used alone or in combination with one or more of the general immune suppressor agents or with other specific immune down regulatory agents to insure or augment the stability of cells tissues or organs transplanted into the recipient subject.

In transplantation both the recipient and donor can have specific immune down regulation. Under this condition cells, tissues and/or organs will be derived from subjects that have been previously been rendered tolerant of the donor through specific immune down regulation and placed in the recipient subject who has also in turn rendered tolerant by specific immune down regulation. This process will eliminate or diminish rejection. This double procedure can be accompanied with various immunosuppressive procedures to enhance the recipient's capacity to support the transplanted cells, tissues or organs.

Still another useful process of the present invention is one that induces tolerance in any subject. Tolerance is induced its a first subject by transferring the cells from a second subject to a first subject wherein SIDR has already been established in the second subject by the transfer of immune cells.

When there is a requirement to establish that tolerization has been conferred upon a subject, this can be accomplished either by directly assessing tolerization by a challenged type of assay or indirectly by measuring some other parameter that is associated with the induction of tolerance. Exemplary direct methods of assessing the tolerization are the *in vivo* introduction of the antigen info the subject and measuring the extent of an immune reaction (as described in the teaching of the present invention where antibody levels to antigens were measured) *or ex vivo* by removing some of the lymphocytes and assessing their ability or potential ability to react to antigen stimulus. Assessment of the extent of the immune reaction to antigen challenge can be carried out by a variety of means well known to those versed in the art. Induction of tolerance can be measured indirectly by surrogate markers that undergo changes in a subject when tolerization has occurred. Exemplary markers are the level of TGFß, and other cytokines (Hancock et al., (1995) Am), Path. 147; 1193-1197, incorporated by reference).

In another aspect of the present invention methods are presented that can be useful in the treatment of diseases that are caused by a pathogen wherein the immune response to such a pathogen plays a significant role in the pathology of such an infection. Immune responses to virus infections, for example, involve CTL activity that acts to clear the virus from the body by killing virus infected cells or releasing appropriate cytokines. Although this response benefits the host in most viral infections, some viruses that present no direct cytopathic effects to the host can produce severe inflammatory disease as a result of the immune response. In viruses such as hepatitis B virus the immune reaction to this virus is believed to be the major cause of hepatocellular damage. The immune response can be strong enough to produce subacute or chronic hepatitis or even acute liver failure, all of which indicate a poor prognosis.

The distinction between the direct effects of the virus and the indirect effects produced by the reaction to the virus has been demonstrated in an *in vivo* model where HBV genomic DNA was transferred into rats (Takahashi et al., Proc. Nat. Acad. Sci., 92:1470 - 1474 (1995)). Although rats are not ordinarily susceptible to HBV infection, in this case infection was established as demonstrated by the presence of viral replication and expression of virus genes in the rat hepatocytes, Also, the infected rat livers showed extensive indications of hepatocellular damage that closely resembled human symptoms of hepatitis B infections. These included elevated levels of glutamic-pyruvic transaminase (a liver enzyme released from damaged hepatocytes) and histopathology indicating hepatocyte death and infiltration by lymphocytes. In contrast to the pathology produced by such an HBV infection in rats, athymic nude rats showed no such pathology, *i.e.*, no signs of hepatocellular damage even though viremia was present and even persisted longer than in the normal rats. (Guidotti, et al., J. Vir 69:6158-6169 (1995))

In another approach, transgenic mice have been produced that contain copies of HBV DNA as part of their genetic complement (Guidotti et al., 1995). These mice contain episomal replicative HBV DNA intermediate and express HBV gene products and release viral particles that resemble those seen in a normal infection. Yet, despite the similarity to an ongoing chronic HBV infection, the livers are functional and show no signs of any defects or damage.

These studies serve to indicate that the absence of an immune response to HBV infection decreases the damaging effects of an ongoing HBV infection.

The present invention provides compositions and methods of use for the treatment of diseases caused by a pathogen that can elicit an immune response that itself is a major contributing factor to the resulting pathology. In contrast to commonly practiced therapeutic procedures for such diseases that attempt to enhance the immunological capacity to resist infection, the present invention takes an opposite approach by the use of methods that can eliminate or suppress the immune response to the pathogen. This invention thus provides a selective suppression of the immune response by tolerization to HBV wherein this can be achieved by viral components that are involved in the induction of the antiviral immune response. These include the surface protein or the viral envelope, the core proteins, the pre S1 and pre S2 proteins, as well as other virus proteins. Such compounds can be provided as their intact natural structure or as fragments thereof wherein they can be produced by chemical synthesis or by the methods of recombinant DNA. Such compounds can be provided in purified, partially purified or crude forms and can be used in intact or partially digested states. Such compounds can be administered in contact with other agents such as adjuvants or delivery systems that could be further complexed or conjugated or otherwise modified to provide for stability or for more efficient administration. Other useful entities for this purpose include dead or inactivated viruses. Such compounds or entities could be administered orally, by intraportal vein inoculation, dominant transfer of tolerance and others. Also useful in the scope of the current invention are antibodies or other reagents that temporarily repress selected segments of the immune system.

While chronic HBV infection is a deleterious disorder usually leading to end stage liver disease, most patients cannot benefit from antiviral agents such as interferon. The present invention can permanently depress the immune system and thereby abrogate any hepatocellular damage while other therapeutic agents may or may not be used to provide treatment for the viral infection itself. Because the majority of the hepatocellular damage is a result of the immune response, and the virus itself is non cytopathic, the viremia should not he harmful. Tolerizatian to eliminate or significantly suppress the pathology associated with an HBV infection can therefore essentially transform a chronic HBV patient into a "healthy" carrier whose response to the infecting HBV is similar to the vast majority of the HBV infected patients who carry the virus for life without the development of any major complications, In these cases the immune system cannot clear the virus, and the patient can be considered as tolerized to HBV. Chronic HBV patients, in contrast, are patients in whom the immune system, as a consequence of attempting to clear the virus, damages virus-infected as well as non-infected hepatocytes. In this case also, transforming of a chronic HBV patient into a "healthy" HBV carrier by tolerizing against the virus can alleviate or even cure the hepatocellular damage.

Tolerization to HBV can also be useful for eliminating or reducing HBV recurrence in patients who have received liver allografts. Currently, HBV recurrence is the major obstacle for liver transplantation in patients with HBV related illnesses: the rate of recurrence in such patients is 20.90% within the first year post transplantation. Infection is considered to result from HBV infected bone marrow and peripheral blood lymphocytes that appear to be major reservoirs for the virus. HBV recurrence in post liver transplantation is usually associated with severe liver injury that is considered to be immune mediated and which normally leads to a rapid deterioration in liver functions and death.

While the exact mechanisms involved in the induction of tumors by the virus are unknown, it appears to involve integration of parts of the virus into liver cells genome as well as involvement of a defective antiviral immune response. Thus, in cases where the immune response to the HBV may have a role in the tumurogenesis of the virus, tolerization to HBV can provide useful benefit to the prevention of development of hepatocellular carcinoma.

The present invention provides compositions and methods of use for therapeutic agents that have antigenic properties wherein such agents can be used without the risk of an unwanted immune response.

Specific immune responses to foreign and infectious agents are recognized as being both necessary and beneficial to the maintenance of a healthy subject, in fact, induction of an immune response to an infectious agent (vaccination) and promotion of an effective immune response in the face of infection are desired and recommended therapeutic regimens. However, in certain instances, an immune response to an infectious agent leads to undesirable consequences such as an autoimmune response or direct or indirect destruction of infected or uninfected cells and tissues. For example, in the case of HIV infection. HBV or HBV infection or rheumatic heart disease, such undesirable immune consequences are observed, In spite of such a problem, it is not recommended to limit or inhibit the immune response directed against the infectious agent. As such, there are no effective therapeutic regimens that address both the undesirable immune consequences as well as depropagation of the infectious agent.

It is another aspect of this invention to overcome the limitions that exist in treating the condition of these infections by uncoupling the immunological response from the propagative aspect of the infection and addressing each one independently. According to the teachings of this invention, the subject (patient) is treated with one or more immunological modulation protocols that would lead to a state of SIDR and GIS or a combination thereof while, if desired or necessary, addressing the propagative aspects of an infection by the use of appropriate compounds directed against the pathogen such as anti-bacterial, anti-fungal or antiviral agents including viral protein inhibitors or viral replication inhibitors.

Although HIV infection is noted for the breakdown of the immune system, there is evidence that the loss of CD4* cells is not due completely to the direct effect of viral infection but there also might be an auto-immune components that is responsible for CD4* cell depletion. This was originally suggested in 1986, (Ziegler, J.L. and Stites, O., Clin Immunol. Immunopathol, 41;305) and support for this concept has continued to accumulate. For instance, although chimpanzees can be infected readily by HIV, there are no signs of progression into AIDS as seen in humans. An important difference that has been noted between HIV infections of humans and chimpanzees are the absence of cytotoxic T lymphocytes (CTLs) in the latter (Zarling,et al., (1990), J. Immunol. 144: 2992-2998. in contrast, infections in humans resulted in the production of CTLs that are capable of lysing uninfected CD4* cells from humans and chimpanzees. Also a correlation between the level of autoantibodies and the level of depletion of CD4* cells has been observed (Muller, C., Kukel, S., and Bauer, R. (1993) Immunology 79: 248-254, incorporated by reference herein).

Thus, even while therapeutic means are employed to block viral replication, there can be a system that maintains ongoing destruction of CD4' cells in such a case, therapeutic benefits are achieved by applying the teachings of this invention. One or more immune modulation protocols is administered to the patient to induce a SIDR or GIS state against the viral antigen or viral antigen complexed with antibody or viral antigen complexed with cell receptors or viral antigen within the cell matrix.

The patient, in such a state will exhibit reduction, inhibition or elimination of an autoimmune response. The patient is maintained on an anti-HIV regimen which includes protease inhibitors and/or inhibitors of viral replication. Such a protocol could be supplemented with the appropriate cytokines (reviewed by A. Fauci, (1996) Nature 384; 529-534, incorporated by reference herein) or antibodies directed against specific T-cells,

In patients with HIV infection, the circulating antibodies do not provide protection to the host. In addition, both the viral antigen and anti-idiotypic antibodies that mimic the antigens, can bind to CD4¹ cells and will interfere with the functioning of these cells and may induce their apoptosis. The component of the HIV particle that is responsible for binding to the CD41 receptor is the viral gp120 protein. Vaccines have been made that are based upon this protein to elicit an antibody response to the HIV (Eron et al. (1996) The Lancet, 348; 1547-1551). No effects were seen, however, in the progression of the disease.

Based on the teachings of this invention, oral administration of HIV proteins and/or components and its complexes and conjugations to the corresponding antibodies or receptors (CD4) or cell membranes containing such all HIV antigen, in appropriate doses and duration will reduce the antibody levels. The patient in this stage will demonstrate reduction or inhibition of the autoimmune response. Furthermore, these patients will show an improved immune competence, The HIV load will be reduced by cotreatment with currently available drugs or by newer methodologies, including genetic antisense therapy. This procedure can be generalized to any virus that produces interfering antibodies during the humora! response to the viral antigens. Viruses that may fall in this category include those viruses that evoke an immune response in response to infection and yet the humoral or cell-mediated immune response seems not to be effective against the spread of the virus. Candidate viruses for this type of therapy include HIV-1, HiV-2 and HTLV-1.

In the present example, viral protein (gp120 or its fragment) is administered to an infected patient as a means of oral tolerization to the HIV antigen. Details of methods of production of a viral protein and administration of proteins in an oral tolerization program are described in the teachings of this invention and in "Oral Tolerance: Mechanisms and Applications" H.L. Weiner and L.F. Mayer, eds. (1996) The New York Acadamy of Sciences New York, N.Y., the contents of which are fully incorporated herein by reference. When the patient still has a functional immune system at this stage of the disease, there should be tolerization to the viral antigen that should diminish the rise in an autoimmune response that could be generated by this protein.

This dual treatment protocol would allow blocking of viral replication and propagation while keeping the autoimmune system dormant. If the patient is in a GIS state during the course of treatment, then, after effective anti-viral propagation therapy, the immune suppression can be released allowing restoration of the immune system,

An aspect of this invention is to use immunological modulation protocols taught by this invention to block or to diminish the number of target cells available to the HIV so that the opportunity of the virus to propagate is diminished, while the patient is maintained on an anti-viral protocol. If a method is used to reversibly block the immune system by reducing the number of T4 cells, or other HIV target cells or their rate of proliferation, by the use of immunomodulation protocols and reagents, or by blocking of the CD4' or other target cell receptors (treatment with ami-CD4* and/or OKT₃ and/or anti-macrophage antibodies e.g., CD14*), the number of available cells for infection by the HIV should be decreased or diminished, allowing a method of increasing the impact of anti-viral therapeutic agents,

Whereas efforts to treat a variety of diseases by the administration of compounds such as proteins and polypeptides can provide effective therapy, useful benefit can be diminished by immune responses to the therapeutic agent, especially when the therapeutic agent is non-native to the patient. The present invention overcomes these limitations and opens pathways to the use of a broad range of such agents. This advantage is achieved by the use of immune modulation as a means of providing for effective *in vivo* treatment with such compounds. Compositions and methods of use are provided herein for transient or prolonged use of such therapeutic agents introduced into the body without the risk of an unwanted adverse immune response.

Therapeutic agents that can be utilized with the compositions of the present invention are non-native, non-viable, have the potential of being immunologically recognized and can perform a biological function wherein they can be synthetic, natural, cloned, modified or an analogue that in the body, indirectly or directly, whether intracellular or extracellular, can perform a biological function or interfere, inhibit or enhance a biological process. Such agents, referred to herein as non-native active compounds, include: enzymes antibodies, ligands, co-factors, hormones, cytokines, lymphokines and factors that induce or inhibit apoptosis and others.

Such non-native active compounds can, in the body, perform a biological function or can interfere with, inhibit or enhance one or more biological functions including such biological functions that are artificially provided (such as by the methods of gene therapy). Non-native active compounds include enzymes such as non-native factor IX that can provide for this missing element in certain types of hemophilia, bacterial bilirubin oxidase that can act to reduce bilirubin concentrations, non-native superoxide dismutase that can remove free radicals in tissues such as cardiac tissue that has been traumatized as a result of myocardial infarction, *E*. *coli* aspagaginase that modifies L-aspagagine in tumors thereby inhibiting tumor growth, bovine adenosine deaminase for treatment of ADA deficiency and polymerases, integrases and other enzymes that can serve as components of a gene delivery construct.

Antibodies can be useful as non-native active compounds wherein they can be monoclonal, polyclonal or wherein they can be intact natural proteins or fragments thereof and can be modified such as in a chimera with one or more other antibodies or proteins. Antibodies useful as non-native active compounds include monoclonal antibodies such as OKT3 that can ablate peripheral lymphocytes for the purpose of combating acute allograft rejection, and antibodies to CD8 cells as a means of controlling the killing of CD4+ cells in HIV-1-infected individuals (U.S. Patent 5,424,066) as well as other antibodies such as anti-CD4, anti-CD8. anti-Thy and anti-NK that can be used to promote skin graft tolerance (Zhao, Y. et al 1996 Nature Med 11: 1211 - 1216). Both the foregoing patent and the article are incorporated herein by reference. Other useful antibodies include those that block histocompatibility determinants in donor organs, antibodies that recognize and bind to certain tissues and organs and can thus be used for radioimaging, and antibodies that bind to certain tissues and organs and that can be modified with certain cytotoxic agents, such as risin, to provide selective cell killing,

Non-native hormones such as estrogens and androgens can provide such useful functions as inhibiting apoptosis. Nan-native cylokines or lymphokines can provide useful benefit.

Such non-native active compounds can be provided in their natural structure or as fragments thereof wherein they can be natural or can be produced by chemical synthesis or by the methods of recombinant DNA. Non-native active compounds can be provided in purified, partially purified or crude forms and can be used in intact or partially digested states. Non-native active compounds can be administered in contact or in concert with other agents such as adjuvants or delivery system that could be further complexed or conjugated with a recipient antibody and/or a receptor, or with a cell matrix. Such compounds could be modified to provide a longer half-life in the body (Maeda, H. et al. 1992 Bioconj Chem 3: 128 -139;. Non-native active compounds can be modified with ligands, such as biotin, in order to provide binding cells (See Example 7 below)

The present invention provides for the administration of non-native active compounds without the risk of an immune response that could diminish the effectiveness of such treatment whether such treatment is transient or whether such treatment is made repeatedly over a prolonged period. The present invention thus provides for the effective biological function of these non-nativo active compounds without interference by the body's immune response. This can be achieved by the usa of immune modulation as provided in this invention wherein it can be used as general immune suppression for transient or short term treatment and/or by tolerizalion, provided by selective immune down regulation, for prolonged treatment. In some cases a combination of two or more such immunomodulation regimens can be advantageous. Such treatments can be applied prior to and/or during the course of administration of non-native active compounds. Thus, for example, in order to prevent the development of unwanted immune responses to non-native active compounds in the early stages of long-term administration, it may be desirable to commence SIDR measures prior to treatment. Alternatively, in the absence ol any prior measures to establish immunomodulation, SIDR measures can be performed simultaneously with administration of a naon-native active compound. In this case, in order to prevent the development of immune responses during the early stages of treatment, general immune suppression could be used during this period. In cases where an individual has a preexisting capability to mount an immune response to a non-native active compound SIDR can commence prior to administration of such a compound. Alternatively, in the absence of any prior measures to establish immunomodulation, SIDR can commence simultaneously with administration of non-native active compound. In cases where one or a very few treatments are administered over a short period of time general immune suppression may be useful without the requirement for tolerization.

For instance, recombinant adenoviruses are being used lay many investigators four somatic gene therapy. (Ali, et al.. Hepalology, 24:3M,A 1996, Gene Therapy 1:367-384; Jaffe et al., 1992, Nat. Genet. 1:372-378). However, the expression of foreign genes delivered by these vectors is of limited duration both because of the episomal mature of adenoviruses (Prevec, et al, J Gen Virol 1989:70:429-434: Horwitz. et al, Virology Raven Press: New York, 1990:1679-1721) and, more importantly, because of the host humoral and cellular immune response (Yang, et al.. Vicol 1995:67:2004-2015). All of the foregoing articles and book are incorporated by reference. Host cytotoxic lymphocytes (CTL) against adenovirus infected cells may clear the adenovirus infected cells, reducing the duration of transgene expression *in vivo* after the initial injection of the virus. Neutralizing antibodies that appear in response to the initial exposure to adenoviral proteins prevent effective gene transfer to hepatocycles upon reinjection of the virus.

Recombinant adenoviruses are generated by insertion of the target gene into the El region of the viral genome, thus disrupting the E1 gene and rendering the virus replication defective (Graham. et al., Methods in Molecular Biology. The HuinamaPress: Clifton New Jersey, 1991:109-128). Attempts have been made further cripple the adenoviral vector by using a virus containing a mutation in the E2a region that results in the expression of temperature-sensitive DNA binding proteins. However, these "second generation" adenoviruses are still able to invoke a polent anti-viral imagine response (Yang. et al.. Nature Genetics 1994:7 362.369; Engellmard, et al., Proc Natl Acad Sci, USA 1994 91:6196-6200.). It is likely, therefore, that the antigenic load in the input recombinant: virus is sufficient to, produce this immune response. Thus other mechanism of modulating the anti-viral immuneresponse need to be sought.

Studies in nude and SCID mice with defects in T or in both T and B cell functions as well as the use of several immunopressed regimens including FK506,cyclosporine and cyclophosphamide have shown that a longer duration of gene expression can achieved with systemic immunosuppression (Han, et al., J Hepatol abstract) 2573A; Dai (1995) supra; Fang et al..Human Gene Therapy 1995:6:1039-1044).However, the applicabillity of these methods in humans is limited because of the general immunosuppressive state they induce in the recipient. Moreover, these method would not allow gene transfer in the presence of preexisting neutralizing antibodies against the virus.

The treatment processes provided herein are particularly useful for treating indications associated with autoimmune disease. Treatments that utilize tolerization for the treatment of autoimmune disorder are often severely limited by the requirement that tolerizing agents should be obtained from the afflicted individual. A further frequent limitation of current procedures, such as for the treatment of autoimmune diseases of the bowel, is the requirement for preventive use, by prior establishment of tolerization [Sharmanov, A. T, et al. (Gastroenterology 106[1994] A772; Neurath, M,F. et al. J. Exp, Med. 183[1996] 2605-2616). While such preventive measures can reduce the severity of autoimmune responses, they are not therapeutic, and thus are of little benefit to afflicted individuals. As described herein, selective immune down-regulation (SlDR) can be utilized to overcome these limitations by providing both for tolerizing agents that can be conveniently derived from allogeneic and xenogeneic sources as well as autologous sources and for their therapeutic use in the treatment of afflicted individuals.

As also disclosed herein, SlDR can be provided to individuals who are free of disease symptoms in order to provide a means of preventing IBD, or such treatments can be used therapeutically for the treatment at the onset of disease symptoms or concurrent with the presence of disease symptoms. SIDR can also be used in combination with other forms of therapy, such as generalized immune suppression as described in this application.

As also provided herein, tolerizing agents that can be utilized for such SIDR can be obtained from a number of sources including, for examples, afflicted individuals as well as from other normal or afflicted individuals, from cadaver tissue or from animal tissues or from other sources that provide compounds that possess useful antigenic and/or immunological properties. Useful compounds for this purpose include diseased or normal tissues that can be derived from the afflicted individual or from other individuals, from cadaver tissue or from animal tissues. Such tolerizing agents can be used as whose tissues or extracts thereof incluùil1g proteins, glycoproteins, peptides or glycopeptides or any combination thereof in , crude or pure forms. Tolerizing agents can be modified by the addition of haptens and/or by proteolytic treatments. Such proteins can also be prepared synthetically or by the methods of recombinant DNA. Such compounds can be administered by oral routes, by injection or infusion into the stomach, or by other methods as described in this disclosure.

The compositions and methods of the present invention can provide for SIDR for the treatment of inflammatory bowel diseases such as ulcerative colitis and Crohn's disease, the principal inflammatory diseases of the gastrointestinal tract. While studies in mice show that symptoms of inflammatory bowel disease can be reduced by oral administration of an antigenic compound (trinitrobenzenesulfonic acid, TNBS) that is responsible far induction of the autoimmune process that leads to IBD, such treatment was administered to healthy, symptom free animals prior to the induction of IBD (Sharmanov, A. T. *et al.,* 1994,Neurath, M.F. *et al.,* 1996). As disclosed herein SIDR can provide for marked decreases in the severity of such symptoms when treatment is administered in a therapeutic mode at or after the onset of disease symptoms. This can be achieved by the administration of antigenic compounds involved in the autoimmune response responsible for IBD or by compounds that possess similar antigenic and/or immunological properties. Such compounds can be administered by oral routes, by injection or infusion into the stomach, the ileum or by other routes all as provided or disclosed herein. Useful tolerizing agents for this purpose include diseased or normal tissues such as colonic tissues wherein such tissues can be derived from the afflicted individual or from other normal or afflicted individuals, from cadaver tissue or from animal tissues.

The use of SIDR to treat IBD can be demonstrated in rat studies by the feeding of proteins extracted from colons of colitis-afflicted rats to rats is whom colitis had been induced by intracolonic installation of trinitrobenzenesulfonic acid. Marked reduction of symptoms was observed when such SIDR was administered to rats at the same time that induction of colitis was administered. Compared to control rats that received no oral tolerization but had been treated to induce colitis, tolerized rats demonstrated marked decreases in the severity of the colitis symptoms of diarrhea, colonic ulceration, intestinal and peritoneal lesions, wall thickness and adema.

The compositions and methods of the present invention also provide for the treatment of autoimmune diseases such as insulin-dependent diabetes mellitus (IDDM). IDDM is characterized by the presence in islet cells of multiple autoantigens (Atkinson, M.A. and McLaren, N. K., J. Clin. Invest. 921199311608-1616) that provide targets for the destruction of the islet cells in the development, progression and maintenance of the diabetic condition. Previous attempts to tolerize to reduce the symptoms of IDDM have utilized orally administered insulin or insulin fragments (Maron, R. et al. in Oral Tolerance. Mechanism and Applications, Edited by H. Weiner and L. Mayer, Vol. 778, pp. 346-357). Tolerization to insulin, rather than to the islet autoantigens and other non-insulin elements that contribute to the autoimmune responses leading to IDDM ignores the essential role of these elements in the onset and maintenance of the diabetic condition. A direct non-insulin-dependent procedure that utilizes cells, tissues or organs or fractions or extracts thereof that are consequential to the disease process can provide effective treatment of IDDM, Such cells, tissues or organs can be obtained from an autologous source, but can be more conveniently obtained from allogeneic or xenogenic sources. Such tolerizing agents can thus include fat cells and pancreatic cells including islet cells, extracts of such cells and tissues including protein preparations. Such proteins or fractions thereof can include sialoglycolipid, glutamate decarboxylase, insulin receptor, 38 kd antigen, bovine serum albumin glucose transporter, carboxypeptidase H, insulin receptors and others.

Compositions and methods for SIDR as provided herein can reduce or eliminate the symptoms of graft versus host disease (GVHD). For such treatment tolerization of the donor against the major histocompatibility and/or other antigens of the recipient can render the donor's immune cells tolerant of the cellular material of the recipient. An example of such induction of immune tolerance as a means of providing for reduction of the symptoms of GVHD is presented herein where such immune tolerance is established in mice by feeding donor mice with recipient lymphocytes that overexpress MHC class II antigens. Such mice, in contrast to untreated mice, demonstrate a decreased response to donor spleen cells (as measured in mixed lymphocyte assays) when GVHD is induced by injection or donor spleen cells. This study is further described in Example 11 in the experimental section that follows next.

A further elaboration of this invention provides for the treatment and prevention of autoimmune conditions such as rheumatic heart disease and glomerulonephritis that can result from infection with the beta-hemotytic group A streptococcus, *Streptococcus pyogenes.* SIDR can be administered to reduce or eliminate the autoimmune responses that result in heart damage or kidney damage. SlDR can be provided by the administration of autoimmunizing antigens in the form of intact and inactivated *S*. *pyogenes* or extracts thereof. Other useful tolerizing agents include cadaver or animal tissue that contains appropriate autoimmunizing antigens. Administration of said tolerizing-agants can be performed as outlined herein as a means of preventing the onset of RHD and GN in healthy individuals or to treat afflicted individuals, SIDR can also be used in combination with other modes of treatment including antibiotic therapy.

This invention provides a process for producing selective immune down regulation in a subject to an infections bacterial agent. In this process, a reagent or a combination of reagents capable of producing selective immune down regulation and comprising a component or components or fragments thereof of such infectious agent is introduced to the subject, thereby establishing selective immune down regulation in the subject. A preferred infectious bacterial agent for use in this process is streptococcus, including particularly the forms of streptococcus that cause rheumatic fever or glomerular nephrites.

Another significant aspect of this invention concerns a transplantation process comprising the steps of establishing selective immune down regulation in a recipient subject to the antigens of a donor comprising any of the members selected from the group consisting of MHC Class I, MHC Class II and histocompatibility factors, or a combination of any of the foregoing. After establishing selective immune down regulation in the subject, the cells, tissue, or organs, or components thereof from the donor is introduced into the recipient.

Another aspect of this invention is a transplantation process comprising the steps of establishing selective immune down regulation in a donor to an antigen or antigens of a recipient. The establishment of selective immune down regulation is carried out by introducing to the donor any of the cells, tissue, organs, or components thereof from the recipient, thereby establishing selective immune down regulation in the donor to the recipient antigen or antigens prior to transplanting into the donor any of the cells, tissue, organs, or components thereof. Thereafter; the cells, tissue, organs, or components thereof from the donor having selective down immune regulation can be transplanted into the recipient. Particularly useful as recipient antigens or antigens are any of the member selected from the group consisting of immune cells, bone, marrow cells and T cells, or a combination of any of the foregoing. The recipient cells, tissue or organs, or components include by way of example any of those members selected from the group consisting of immune cells, bone marrow cells and T cells, or combinations thereof.

In the transplantation process just described, the donor antigen can preferably comprise any of the members selected from the group consisting of MHC Class I, MHC Class II, and histocompatibility factors, or any combination of any of the foregoing.

This invention also provides a process for treating Crohn's disease in a subject. In this treatment process, selective immune down regulation in the subject to inflamed intestine indicative of Crohn's disease is established by introducing into the subject components, cells, tissues or organs derived from allogeneic sources, xenogeneic sources and autologous sources, or an immunologically equivalent, as explained elsewhere in this disclosure.

Another important aspect of this invention is a process of treating diabetes in a subject by establishing in the subject selective immune down regulation to non-insulin epitopes. Such non-insulin epitopes can comprise any of the member selected from the group consisting of pancreatic cells, insulin receptors, fatty cells and liver cells, or component thereof. These epitopes are derived from allogeneic sources, xenogeneic sources and autologous sources, or an immunologically equivalent, as explained elsewhere in this disclosure.

The examples that follow are given to illustrate various aspects of the present invention. Their inclusion by no means is intended to limit in any way the scope of this invention as more particularly set forth in the claims.

### DESCRIPTION OF THE PREFERRED EMBODIMENTS

### EXAMPLES

### Examples 1 Establishment of Oral Tolerance to Recombinant Adenovirus Antigens

Enteral exposure to foreign antigens has been shown to induce antigen specific tolerance by clonal inactivation of antigen specific T cells or by the induction of regulatory cells secreting factors that suppress the generation of antigen-specific effector cells (Weiner, et al., 994, Proc. Natl. Acad. Sci. USA 91:10762-10765; Vandenback, et al., J. Immunol. 153:852-9 (1994); and Hirahara, et al., J. Immunol, 154:6238-6245 (1995)). Therefore, in this example, high dose and low dose tolerizing regimens were used and it was demonstrated that induction of oral tolerance to adenoviral antigens can be used to abrogate the host anti-adenoviral immune response in a model system that employs Gunn rats, a strain that lacks hepatic bilirubin uridine-diphosphoglucuronate glucuronosyltransferase- 1 (BUGT.).

*Animals:* Inbred Gunn and congenic normal Wistar RHA rats were bred and maintained in the Special Animal Core of the Marion Bessin Liver Center of the Albert Einstein College of Medicine. The rats were maintained on standard laboratory chow and kept in 12 hr light/dark cycles.

*Plasmids:* pJM17 was kindly provided by Dr. F.L.Graham, McMaster University, Hamilton, Canada.

***Generation of recombinant adenovirus:*** Two recombinant adenoviruses. Ad-hOUGT, and Ad.LacZ expressing human bilirubin-UGT, and *E. Coli* b-galactosidase, respectively, were generated as described previously (Takahashi (1996) supra). In brief, transcription units consisting of the promoter and enhancer sequence for the immediate early gene of cytomegalovirus (CMV), the structural region of human BUGT, or *E. Coli* b-galactosidase, and the polyadenylation signal from bovine growth hormone, were recombined into the E1 region of human Ad-5 to produce replication-defective "first generation" adenoviruses. For large-scale preparation, the recombinant adenoviruses were grown on 293 suspension cells and purified from cell lysates by two consecutive CsCl density gradient centrifugations, and stored in 30% glycerol at -20°C. Virus was dialyzed overnight at 4°C against an isotonic solution containing 135 mM NaCl, 5 mM KCl, 1 mM MgCl₂, 10 mM Tris HCl, pH 7.4, and 10% glycerol, and sterilized by filtration through 0.45 um filters before use (Horowitz (1990) supra).

***Preparation and administration of viral protein extract:*** The CsCl gradient supernatant, containing major adenoviral structural proteins, mainly liber, hexon and penton, was collected and the protein concentration was determined (Horwitz, et al., Virology 39: 692-694 (1969). Mazel, et al., Virology 36:126-136 (1968)), Under ether anesthesia, a polyethelene cathether (PE10) was inserted into the stomach through a midline incision. The tube was advanced into the duodenum and affixed to the stomach wall. The other end of the catheter was exteriorized at the dorsal aspect of the neck by subcutaneous tunneling. Each of the rats was kept in a separate cage throughout the study. The protein extracts were introduced through the catheter every other day for 21 days to total of 11 doses).

**Table 1: Experimental groups:**

| **Group** | **Antigen fed and dose:** | **Recombinant virus injected on first injection** | **Recombinant virus injected on second injection** |
|---|---|---|---|
| A | adenoviral proteins: 1 mg per rat per day | Ad-hBUGT, | Ad-hBUGT |
| B | adenoviral proteins: 1 mg per rat per day | Ad-hBUGT, | Ad-LacZ |
| C | bovine serum albumin : 1 mg per rat per day | Ad-hBUGT, | (C1) Ad-hBUGT, (5 rats) |
| D | None | Ad-hBUGT, | ( C2) Ad-LacZ (5 rats) Ad-hBUGT, |
| E | adenoviral proteins: 50 mg per rat per day (n=5), or 100 mg per rat per day (n=5) | Ad-hBUGT, | Ad-hBUGT, |

***Ad-hBUGT, and Ad-LacZ injection into Gunn rats:*** Five groups of Gunn rats, consisting of 10 animals in each group, were studied. Groups A and B included rats that were fed with adenovirus protein extract at a dose of 1 mg/rat every other day followed by two injections of Ad-hBUGT, (5×10⁹ p(u) on days 1 and 98 (Group A), or Ad-hBUGT, on day 1 followed by Ad. LacZ on day 98 (Group B). Two groups of rats were used as controls: Group C received bovine serum albumin 1 mg/day and then received viral injections as described for Group A (5 rats, group C1) or for Group B (5 rats, group C2). Group D did not receive any oral proteins and was injected with Ad-hBUGT, similarly to Group A (Table 1). In order to evaluate the mechanism of the tolerance, and distinguished between induction of suppressor cells and clonal inactivation Group E rats were fed with 50mg/day (5 rats), or 100 mg/day (5 rats) of the viral protein extract (high dose regimen), followed by two injections of Ad-hBUGT.

### Evaluation of immune tolerance

*Liver histology:* For evaluation of the degree of hepatic inflammation, liver biopsies were performed one week after the second injection in 2 rats from each of the treated groups and kept in 10% formaldehyde. Paraffin sections were then stained with hematoxylin-eosin according to standard procedures. The sections were graded for hepatic inflammation as follows: Grade 0: normal; Grade 1: mild periportal or focal lobular lymphocytic infiltration; Grade 2: extension of lymphacytic infiltration into the lobules and "piece-meal necrosis"; and Grade 3: disruption of the lobular architecture by "bridging necrosis" and extension of lymphocytic infiltrates from portal to central, portal to portal and central to central zones.

***Serum alanine amino transferase (ALT) levels:*** As a measure of the degree of hepatic inflammation. ALT levels were qualified using a commercially available kit (Sigma, St Louis, MO).

***Neutralizing anti-adenoviral antibodies:*** Anti-adenoviral neutralizing antibodies present in the sera of treated rats were measured on days 28, 78. 112, and 196 in all rats that received Ad-hBUGT₁ injection. 293 Cells were seeded at a concentration of 3×10 ⁴/well in 96 well plates, and cultured until 90% confluency. Ad-LacZ was diluted in cell culture medium to give 3×10⁶ plu /10ml. Serum samples were heat inactivated at 55ºC for 30 min and diluted in medium in twofold steps. 100 ml of each serum-dilution was mixed with 6×10⁶ plu of the recombinant virus, incubated at 37°C for 90 minutes, and applied to the nearly confluent 293 cells for 10-14 hours. The supernatant containing serum and virus was then replaced by RPMI medium with 10% FCS for 18 hours. Cells were fixed and stained for h-galactosidase expression. In the absence of neutralizing antibodies all of the cells stained blue. The neutralizing antibody titer for each serum sample was reported as the highest dilution at which less than 25% of the cells stained blue. ***Cytotoxic T lymphocyte assay:*** Two rats from each group were studied on days 28, 78, 112 and 196. Spleens were removed under anesthesia from each of two rats at each time point, and the animals resutured. The organs were gently disrupted using a rubber policeman. Red blood cells were removed using lysis buffer containing 0.17M NH₄Cl at pH 7.4 (1 ml/spleen) for 2 minutes. Lymphocytes were spun down and plated at 5 x 10⁷ cells per 5 ml in RPMI medium with 10% FCS. Cells were then restimulated with the recombinant adenovirus AdhBUGT, (1-10 pfu/cell) for 4-5 days. Adenovirus infected primary hepatocytes, harvested by collagenase perfusion of the liver (Seglen, Methods in Cell Biology. 1976)), were used as target cells for the effector lymphocytes and were plated on collagen coated 6 well plates in Chee's medium (2×10⁸ cells/well). Stimulated effector cells were harvested, counted and added to the primary hepatocyte cultures at a ratio of 50-100:1 and incubated at 37°C for 5 hours. Hepatic cell lysis was measured by collecting the medium and measuring alanine aminotransferase (ALT) levels using a commercially available kit (Sigma, St Louis MD) with the following modifications: the ratio between reagent and test medium was changed from 10:1 to 1:1, and the reaction time before the first spectrophotometric reading was 90 seconds, followed by a reading every 30 seconds up to 5 minutes. ALT levels were then calculated according to the manufacturers' formula and expressed in international units. Background ALT levels were determined by measurements of the ALT levels in the supernatants of dishes containing adenovirally infected hepatocytes and lymphocytes from naive rats. CTL activity was expressed in IU of ALT average from 6 wells after subtraction of background levels.

### RESULTS

### Evaluation of Immune tolerance

***Liver histology:*** Liver biopsies from two rats in each group examined 24-72 h after the second injection showed minimal or no periportal or lobular lymphocytic infiltration in recipients that were tolerized by enteral administration of adenoviral proteins (group A). In contrast, a severe inflammatory reaction (grade 3) was observed in liver specimens taken from rats that were given BSA or no protein prior to the injection of the virus (groups C, D) and rats that received high doses of adenoviral antigens (group E) (not shown).

***Serum ALT levels:*** In the group A rats that were tolerized with adenoviral proteins, serum ALT levels increased only minimally after each of the three injections (96-110 IU; normal levels before any manipulation were 60-75 IU). In groups that received BSA or no protein (C and D), ALT levels increased to 168 IU after the first injection, and to 212 IU after the second injection.

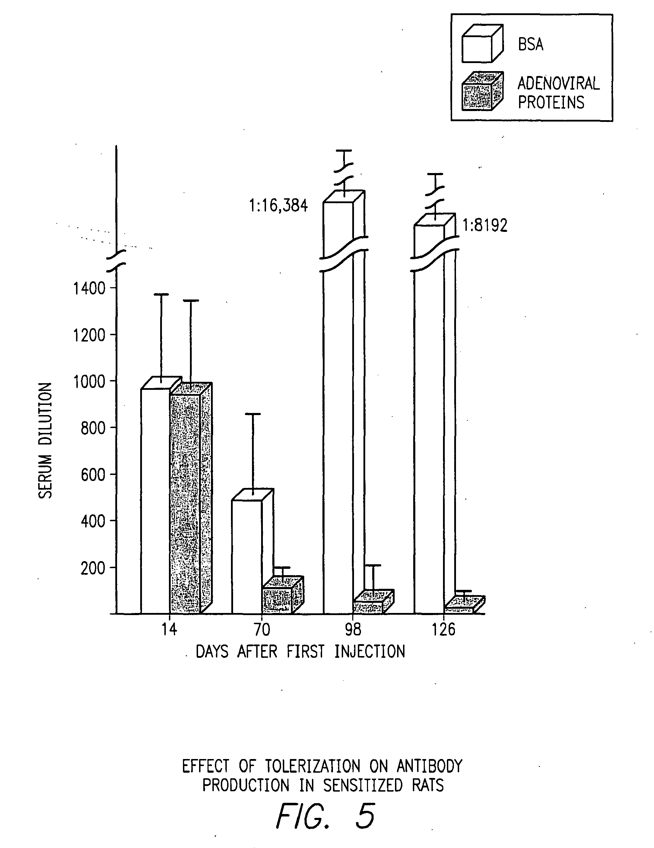

***Neutralizing antibodies:*** After injection of Ad-hBUGT₁ in rats that had received BSA or no proteins (Groups C, and D, respectively) high titer ( > 1 :2816) antibodies appeared during the first month. In contrast, in the tolerized rats (Group A), neutralizing antibodies were undetectable in 80% of the recipients. The remainder exhibited low titers of the antibody (<1:16) (Fig. 5). Rats that developed the low titer antibodies had similar hypobilirubinemic responses to the second injections of Ad-hBUGT, as did the rats that had no detectable antibodies.

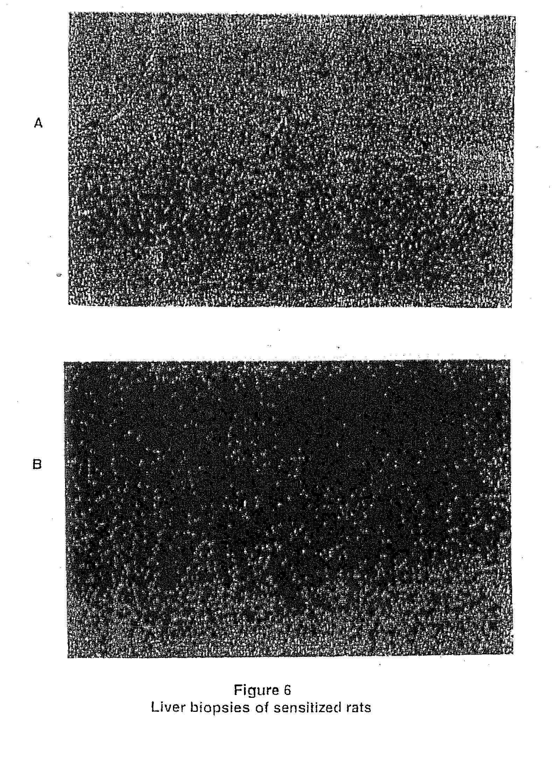

***Cytotoxic T lymphocyte response:*** Cytotoxic T cells were tested against adenovirus infected rat hepatocytes four times throughout the study. Measurement of the amount of ALT released from the hepatocyte targets into the media was used to assess the CTL response. ALT levels in the media were below 80 IU in all tolerized recipients (groups A and B1, but exceeded 150 IU in non-tolerized rats (groups B and C) (Fig. 6 ).

***Effect of antigen dose:*** To evaluate the relationship between the dose of adenoviral proteins and induction of tolerance. 10 rats in group E were fed with the viral proteins at higher doses (50-100 mg/day). In these rats serum bilirubin levels and HPLC analysis of pigments excreted in bite indicated that the second recombinant adenoviral injection failed to achieve gene expression or a metabolic effect. The anti-adenovirus immune response in this group was similar to that in rats that were administered BSA or no protein at all (groups C and D). Thus although no evidence for tolerance was observed using higher doses of the antigen, the administration of low dose feeding of adenoviral proteins markedly inhibited both humoral and cellular host immune response to the recombinent adenovirus containing the human BUGT gena.

High dose feeding, which has been shown to induce energy or deletion of antigen reactive T cells, was found to be ineffective. In contrast, oral tolerization with low dose feeding of adenoviral protein extracts, markedly inhibited both the humoral and cellular host immune response to the recombinant adenovirus containing the human BUGT gene.

### Example 2 Oral Tolerization to Recombinant Adenovirus Prolongs Expression Time and Permits Readministration

The animals used in Example 1 were also used to evaluate the effect of an oral tolerization regime on the length of expression from recombinant adenoviruses

### Assessment of transgene expression.

***b-Galactosidase expression**:* Gunn rats from groups that received AdhBUGT as the first injection and Ad-LacZ as the second injection with (group B) or without (group C2) previous administration of adenoviral proteins underwent liver biopsies. Specimens were frozen in Tissue Freezing Medium (Triangle Biomedical Sciences, Durham, NC), in a dry ice cooled methyl butane bath. Frozen Cryostat sections (10 um) were fixed for 5 minutes at room temperature in freshly prepared 1 % glutaraldehyde in PBS. β-Galactosidase activity was detected by immersing the section into 5-bromo-4-chloro-3-indol-b-galactopyranoside (X-Gal) staining solution (5mM K₄FeCN, 5mM K₃FeCN, 1mM MgCl₂. containing 1 mg of X-Gal per ml for 8-15 hours at 37° C. Sections were briefly counterstained with eosin, then dehydrated and mounted.

***DNA analysis using PCR**:* To detect the presence of the human BUGT gene in the host liver, DNA was extracted from RNase treated tissue homogenates. Two rats from each of the experimental groups A and E, and the control groups C1 and D. were tested 3 days after the second Ad-hBUGT injections. DNA was subjected to amplification by polymerase chain reaction (PCR) using primers (sense: 5'AAGGAAAGGGTCCGTCAGCA 3' from nt 141 to nt 160. antisense: 5' CCAGCAGCTGCAGCAGAGG 3' from nt 441 to nt 4621 designed to amplify a 321-bp segment of the the unique exon 1 (exon 1 *1) of the human BUGT gene, PCR amplification was performed using the following protocol: 94°C for 30 sec, 58°C for 30 sec, and 72°C for 1 min x 30 cycles.

***Expression of human-BUGT protein**:* For determination of the expression of hBUGT liver specimens were taken from two rats in experimental Group A and control Group C1 live days after the second viral injection. Tissue homogenates (200 mg/ml) were prepared in 0.25 M sucrose/10 mM Tris-HCl, pH 7.4 using a glass homogenizer fitted with a motor-driven teflon pestle. For immunobiol analysis, proteins (100 mg/lane) were resolved by electrophoresis on SDS-polyacrylamide (7.5%) gets and electroblotted to nitrocellulose membranes. The membranes were probed with a monoclonal antibody WP1 directed at the common carboxyterminal domains of UGT isoforms expressed by hBUGT, followed by peroxidase conjugated goat anti-mouse IgG F'ah fragment antibody (Sigma, St. Louis, MO) and substrate (Pelers, et al Gastroenterology 93: 162-169 (1987); Towbin, et al., Proc. Natl. Acad. Sci. USA 76:4350-4354 (1979). Equal protein loading in all lanes was assured by performing the electroporesis on an identical SDS-polyacrylamide gel and staining the protein bands with Coomassie brilliant blue.

***Assay for BUGT, activity towards bilirubin:*** The enzyme assay was performed on homogenates of liver specimens from two rats from each experimental group that received Ad-hBUGT injection (A, E, C1 and D), 20 days after the first and second injection, and from all other rats at the termination of the experiments. The assay method was as previously describe, using 80 mM bilirubin as the eglycone (Trotman, et al., Anal Biochem 121:175-180 (1982); Roy Chowdhury, el al., Hepatology 1:622-627 (1981),

***Determination of serum bilirubin levels:*** Serum bilirubin levels were measured according to Jendrasik and Grof in all groups every 10-14 days throughout the study period (Trotman (1992) supra).

***Bile pigment analysis:*** For definitive demonstration of bilirubin glucuronidation in selected rats, bile was collected through a polyethylene bile duct cannula and bilirubin glucuronide excreted in bile was analyzed by HPLC using a uBondapak C-18 column (Millipore-Waters, Millord, MA) as described previously (Roy Chowdhury (1982) supra). Bile was analysed in two rats from experimental groups that received adenoviral proteins at 1 mg/day (group A), 50 mg/day or 100 mg/day (group E) BSA mg/day (groups C1 or no protein at all (D), 20 days after the first and second injections of the recombinant adenovirus. All other rats had bile pigment analysis at the termination of the experiments.

### RESULTS

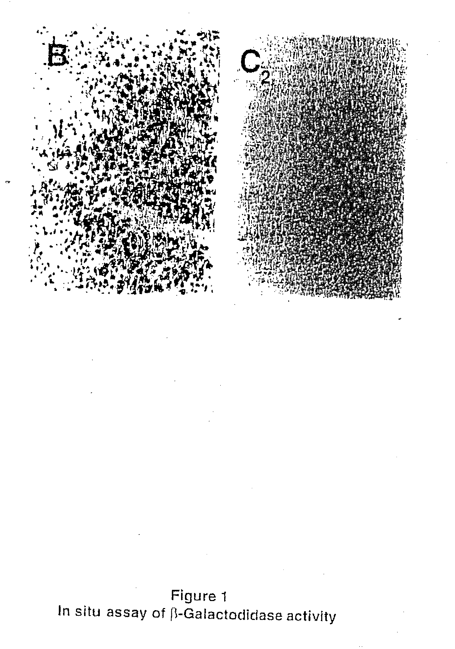

**Expression of b-galactosidase activity:** For histochemical staining, liver biopsies were performed. 7 days after Ad-LacZ injection from liver specimens of Gunn rats that received Ad-hBUGT as the first injection and Ad-LacZ as the second injection with (group B) of without (group C2) prior administration of adenoviral proteins. Biopsies were performed on two rats in each group. Histochemical staining of cryostat sections (10 mm) showed that the great majority of hepatocytes stained positive (or b-gatactosidase activity after the infection in rats that had been administered the adenoviral proteins (group B). while only 5% of hepatocytes stained positive, in livers from rats that were given BSA (group C2) (Fig. 1).

### Expression of h-BUGT gene after recombinant adenoviral injection into Gunn rats.

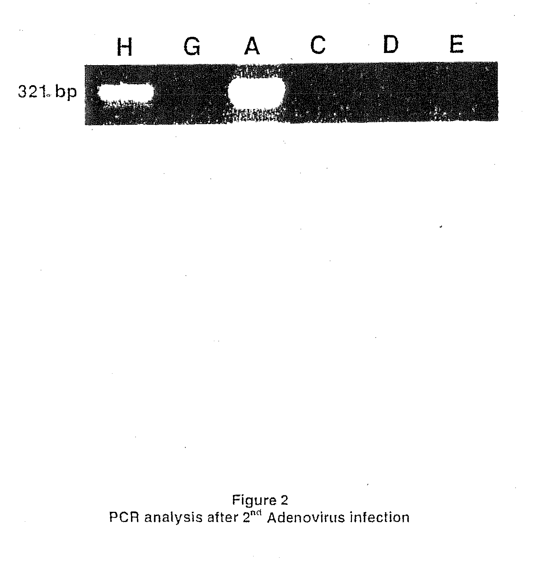

***DNA analysis using PCR:*** Presence of hBUGT DNA in the liver of Gunn rats that received AdhBUGT with (group A) or without (groups C1 and D) without prior enteral administration of adanoviral proteins was evaluated by PCR after the second AdhBUGT injection. A DNA fragment of 321 bp was seen only in rats from group A, while both control groups C and D, were negative. Normal human liver and liver from an untreated Gunn rat were used as positive and negative controls, respectively (Figure 2)

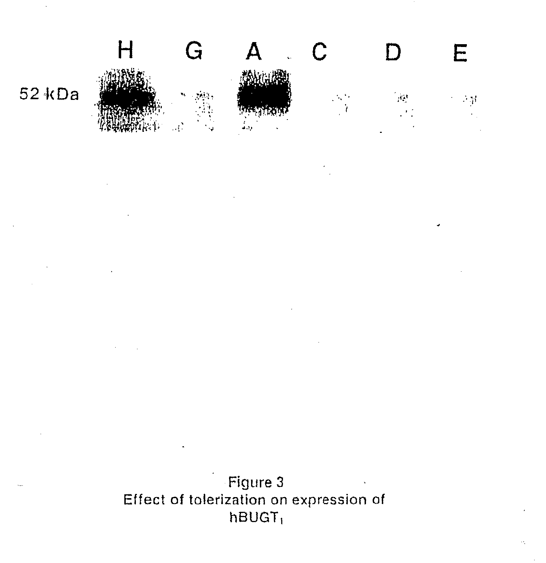

***Expression of human-BUGT protein:*** Liver specimens were collected from two rats in groups A and C1, 5 days after the second Ad-hBUGT injection. Immunoreactive 52 kDa bands, corresponding to hBUGT were observed in Gunn rats that were given two infections of Ad-hBUGT after the administration of adenoviral antigens (group A) but not in the group that received the virus injections after enteral administration of BSA (group C) (Fig. 3). Normal human liver and untreated Gun rat livers were used as positive and negative controls respectively.

***BUGT, activity in vitro:*** UGT activity toward bilirubin was undetectable in untreated Gunn rats. In homogenates of normal human specimens obtained from cadaver donor organs, the BUGT activity was 78+26 nmol/mg liver weight/min: (mean+SEM, n=61. In liver homogenates from two rats that received Ad-hBUGT, injections after enteral administration of adenoviral proteins (group A), bilirubin-UGT activity was 80 and 85 nmol/mg liver wet weight/min, 20 days after the first Ad-hBUGT injection, and was 88+20 nmol/mg liver wet weight/min (mean +SEM. n=5) 20 days after the second injection. In the BSA-led rats, (groups C1 and D), bilirubin-UGT activity was undectectable after the second injection of ad-hBUGT.

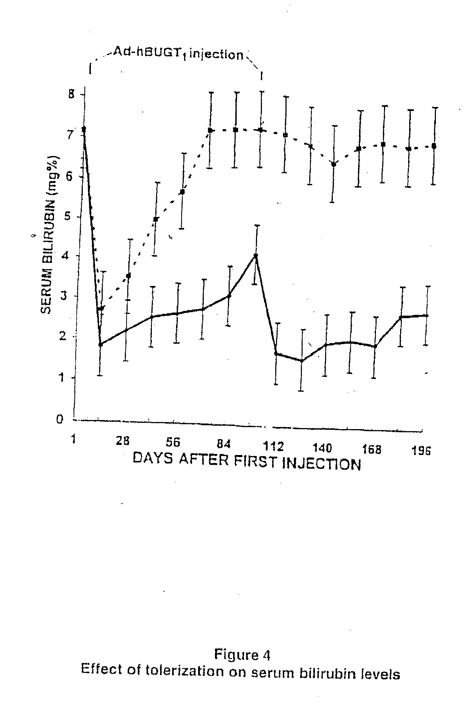

***Serum bilirubin levels:*** Bilirubin levels were measured every 10-14 days. A marked decrease in bilirubin levels occurred after each Ad-hBUGT injection in Gunn rats that were tolerized by the administration of adenoviral proteins (group A), with levels reaching as low as 1.83 , and 1.78 mg/dl after the first and second injections, respectively (Fig. 4). Bilirubin levels remained low for over three months after each injection, and then increased gradually. In contrast, in BSA-fed Gunn rats (Groups C and D) the first Ad-hBUGT injection reduced serum bilirubin levels to 2.73 mg/dl for only 4 weeks, followed by a progressive increase to preinjection levels. Subsequent Ad-hBUGT injections had no effect on serum bilirubin concentrations in these groups.

***Bile pigment analysis:*** HPLC analysis of bile collected from two rats from AdhBUGT-treated rats (groups A, C1, D and E) 20 days after the first injection of AdhBUGT showed excretion of bilirubin mono and diglucuronide. The two glucuronides accounted for more than 95% of the bile pigments, less than 5% being unconjugated bilirubin. This profile was similar to that seen in normal Wistar rats. A similar pattern was seen in rats tolerized by enteral administration of mg/day adenoviral proteins (group A) 20 days after the second Ad-hBUGT injection. In rats that received BSA (groups C) , no protein (group D) or high doses of adenoviral proteins (50-100 mg/day, group E), bile pigment analysis after she second Ad-hBUGT injection did not show significant amounts of conjugated bilirubin in the bile. Gunn rats injected with Ad-LacZ did not excrete bilirubin glucuronides in bile. Chromatographic profiles in bile from these rats resembled that from untreated Gunn rats.

### DISCUSSION

Oral tolerization prolonged the transgene expression, as shown by the longer duration of the hypobilirubinemic effect. However, the effect was not permanent, as indicated by the gradual increase of serum bilirubin levels between day 14 and 98 after the first Ad-hBUGT₁ injection. A similar decay of transgene effect was seen after induction central tolerance to recombinant adenoviruses induced by injection of the virus during the newborn period (Takahashi supra). The decline of transgene effect seems to have resulted from the degradation of the episomal adenoviral DNA, rather than the loss of tolerance, because there was no antiviral CTL activity in the host during this period.

In conclusion, this example shows the potency of low dose oral viral antigen administration in down-regulating the antiviral immune response., In addition, this example demonstrates that after the activity of the desired gene product is diminished, the recombinant adenovirus can be re-administered without an immune reaction. This method is useful in clinical practice in order to tolerize the host to a useful recombinant adenovirus, and opens the possibility of providing effective long-term gene therapy for inherited metabolic diseases using these vectors,

### Example 3 Oral Tolerization to Recombinant Adenovirus is Stable

To determine whether oral tolerization results in long-term tolerance, Gunn rats were tolerized by oral administration of adenoviral proteins as described in example 1. Eight months after the tolerization, the rats were injected with Ad-hBUGT₁. This did not result in either humoral or CTL response. These results indicate that even in the absence of the presence of the tolerizing antigens, the tolerization is long-lived.

### Example 4 Establishment of Oral Tolerization to Pre-Existing Immunoreaction

The previous examples have demonstated that long-term adenovirus-directed gene therapy, can be achieved by tolerizing the host specifically to antigens of the recombinant adenovirus by oral administration of adenoviral antigens. This permits long-term transgene expression without systemic immunosuppression. Some serotypes of adenoviruses, including adenovirus type 5, the serotype in which most gene therapy vectors have been constructed, commonly infect humans. Therefore, many adult humans have preexisting neutralizing antibodies and cytotoxic lymphocytes against adenoviruses, which would pose an obstacle to the clinical application of these vectors (Weiner (1994) supra; Vandenback (1994)supra).

The present example demonstrates that oral tolerization, in addition to preventing the appearance of host immune response, can also reduce preexisting antiadenoviral antibody titers and cytotoxic lymphocyte response. The results demonstrate, for the first time, that by enteral administration of the major adenovital structural proteins into preimmunized rats it is possible to reduce the preexisting antiadenoviral immune response to a point at which it possible to express the transgene by intravenous injection of a recombinant adenovirus.

Details of the protocols for the tolerization viral administration and analysis of immunological effects were as described for Example 1. Details of the protocol for assessment of gene expression were as described for Examples 2

Ad-hBUGT, injection into Gunn rats. Three groups of Gunn rats, two consisting of 10 animals in each (A and C). and one with five rats [B], were studied (Table 1). All rats were injected with the recombinant virus Ad hBUGT 15x10⁹ and the induction of high titers of anti-adenovirus neutralizing antibodies was verified as described below. Groups A rats were fed with 10 doses of adenoviral-proteinextracts (1mg every other day) starting on day 40 after the first adenovirus injection. Rats in Group B were fed with 5 doses of that adenoviral proteins starting on day 10. Group C rats (control received 10 doses of bovine serum albumin (1 mg every other (day) starting en day 40. Rats in all groups received a second injection of Ad-hBUGT, (5 x 10³ plu) on day72

**Table 2: Experimental groups:**

| Group | (n) | Antigen fed and dose | Number of doses | Days of feeding (after first virus injection) |
|---|---|---|---|---|
| A | 10 | Adenoviral proteins | | |
| | | 1 mg per rat daily | 10 | 40-58 |
| B | 5 | Adenoviral proteins | | |
| | | 1 mg per rat daily | 5 | 10-18 |
| C | 10 | 1 mg bovine serum | | |
| | | albumin per rat per day | 10 | 40 58 |

### Assessment of transgene expression.

***DNA analysis using PCR_{:}*** To detect the presence of the human BUGT, gene in the host liver, DNA was extracted foam RNasa-treated tissue homogenates as described previouslyin Examples 2. Two rats from each group were tested 5 days after the second Ad-hBUGT) infection. RNA was subjected to amplification by polymerase chain reaction as described in Example 2

*Expression of human-BUGT, protein:* For determination of the expression of hBUGT, liver specimens were taken from two rats in experimental Groups A and B, and control Group C five days after the second viral injection. Tissue homogenates were processed and analyzed as described in Example 2 *Determination of serum bilirubin levels-* Serum bilirubin levels were measured every 10 .14 days throughout the study period as described in Example 2

*Bile pigment analysis:* For definitive demonstration of bilirubin glucuronidation In selected rats, bile was collected and analyzed as described in Example 2. Bile was analyzed in two rats from each group, 14 days after the second injection.

### Evaluation of Immune tolerance.

*Antiadenovirus antibodies by enzyme-linked immunosorbent assay (ELISA);* Detection of anti Adenoviral antibodies by ELISA was performed by coating 96 well plates with 1x10⁸ particles per well of Ad-hBUGT in PBS at 4°C overnight. The wells were washed five times with30 mM sodium phosphate containing 150 mM NaCl (PBS) and 1% Tween-20, blocked with 3% BSA in PBS. washed again and incubated for 2 hours with serial dilutions of the sera (in 1% BSA) at 37°C. antibody levels were maasured after 0.1 M mercapthoethanol incubation of the sera for 1 hour at 37°C. The wells were washed and incubated with 100ml of 1:1000 dilution of alkaline phosphatase-conjugated goat anti-rat IgG (Bethyl Laboratories. Montgomety, TX1, [or 2 hour at 37°C. After washing, the wells were incubated with substrate (104 Phosphate Substrate, Sigma Diagnostics. St Louis), and read at 405 nm in an ELISA reader. Two negative control sera from naive Gunn rats. were includes in each plate. End point titers were expressed as the reciprocal of the highest dilution that produced an absorbance at least two-fold sister than that observed with negative controls. Sera of rats from all groups were tested on days 0, 14, 70, 98 and 126, after the first injection.

*Liver histology:* For evaluation of the degree of hepatic inflammation, 10% lormaldehyde-fixedliver biopsies were performed one week after the second injection in 2 rats from each group Parallin sections were stained with hematoxylin-eosin according to standard procedures. The sections were graded for hepatic inflammation as follows: Grade 0: normal: Grade1: mild periportal or local lobular lymphocytic infiltration; Grade2: extension of lymphocytic infiltration into the lobules and "piece-meal nacrosis"; and Grade 3: disruption of the lobular architecture by "bridging necrosts" and extension ol lymphocytic infiltrates from portal to central, portal to portal and central to central zones.

***Cytotoxic lymphocyte response:*** Two rats from each group were studied on days 50 and 98. Spleens were removed under anesthesia from each of two rats at each time point and the animals resutured. Subsequent analysis was as described in Example

### RESULTS

### The effect of oral tolerization.

***Antiadenovirus antibodies:*** Serum IgG anti-adenovirus antibodies were examined by ELISA on days 0, 14, 70, 98 and 126 after the first injection, in all rats from groups A, B. and C.Antiadenovirus antibodies appeared in all three groups after the first injection, with titers rising to a peak of 1:2¹⁰ at day 14 (Fig 2). However after enteral administration of 10 doses of adenoviral antigens (Group A1, the antibody titers decreased to 1:2' at day 70 (Fig 5, solid bar), in the BSA-treated controls (Group C), there was only a slight decrease (1:2⁹) in the anti-adenovirus antibody-titers(fig.5,open bars). Following the second injection of Ad-hBUGT, on day 72 after the first injection, there was a boosting of antibody titers (1:2¹⁴) in the control group. In contrast the antibody titers progressively decreased in the orally tolerized rats (Group A, Fig 5, solid bars) despite the second injection of the recombinant adenovirus. Similar results were seen in the tolerized Group B, in which only 5 doses of the adenoviral proteins were administered between day 10 and 18 after the first adenovirus injection (not shown in Fig. 5). Twenty six and 54 days after the second adenovirus injection, the difference in antibody titers between the tolerized and non-tolerized groups were statistically significant (p<:0.005. by Student's T test)

***Cytotoxic lymphocyte response:*** Cytotoxic T cells against rat hepatocytes infected with adenoviruses were tested twice (day 50 and day 98 after the first Ad-hBUGT injection during the study, in two rats from Groups A and C. ALT activity released in the media from the hepatocyte targets was used to quantify the CTL response. ALT levels in the media were 449 and 409 IU in GroupA. and 421 and 533 IU in Group C 50 days alter the first virus injection. Twenty six days after oral tolerization. ALT activity released in the media decreased to 166 and 142 IU in Group A, even though these rats had received a second dose of Ad-hBUGT, on day 72. In the BSA-treated controls (Group C) CTL activity as reflected by ALT activity in the media continued to her high (399 and 476 IU).

*Liver histology:*Liver biopsies from two rats in each group examined 24-72 h after the second injection showed minimal or no periportal or lobular lymphocytic infiltration in group A. In contrast severe inflammatory reaction (grade 3) was observed in liver specimens taken from group C (Fig. 6)

### Expression of h-BUGT gene after the injection of Ad-hBUGT, into Gunn rats.

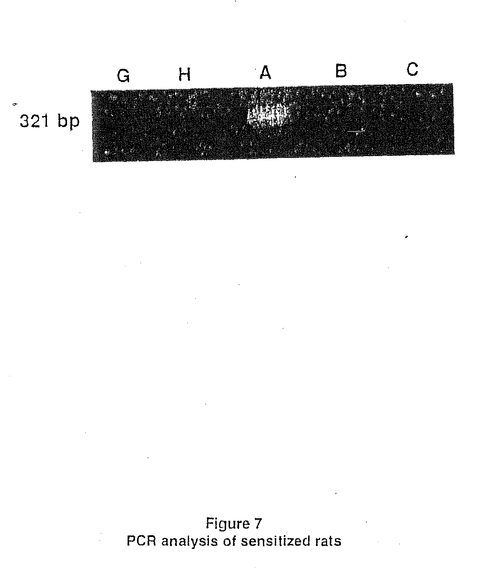

*DNA analysis using PCR:* Presence of human BUGT, DNA in the liver of Gunn rats from groups A and B was tested by PCR after the second AdhBUGT, injection. The expected 321-bp) amplicon was seen only in rats from groups A and B, while Control group C was negative. Normal human liver and liver from an untreated Gunn rat were used as positive and negative controls. respectively (Fig 7).

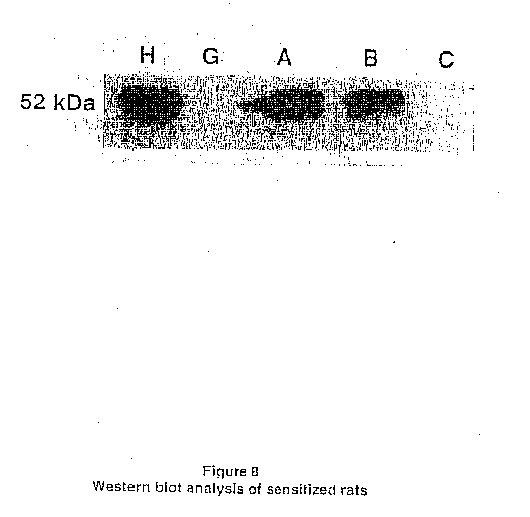

*Expression of human·BUGT,protein:* Liver specimens were collected from two rats in groups A. B, and C, 5 days after the second Ad-hBUGT, injection immunoreactive 62 kDa bands corresponding to hBUGT, were observed in the treated Gunn rats in groups A and B following the second injection, but not In rats from the control group C (Fig. 8). Normal human liver and untreated Gun rat livers were used as positive and negative controls respectively.

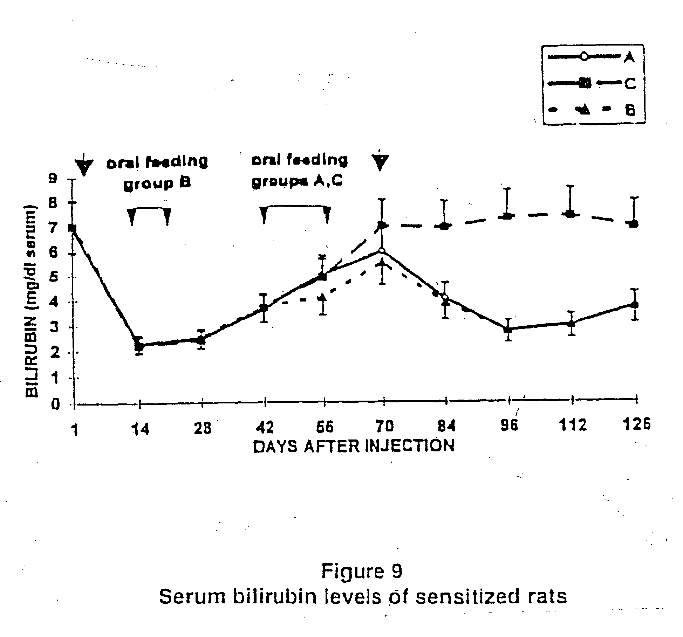

Serum *bilirubin* levels: Bilirubin levels were measured every 10-14 days. A marked decrease in bilirubin levels occurred after each Ad-hBUGT, injection in groups A and B. with levels reaching as low as 2.2, and 2.78 mg/dl after the first and second injections, respectively (Fig. 9). In contrast, in untolerized Gunn rats (Group C), the second Ad-hBUGT, injection had no effect on serum bilirubin concentrations.

*Bile pigment analysis:* HPLC analysis of bile collected from two rats in groups A, B and C, twenty days after the second injection of Ad-hBUGT, showed excretion of bilirubin monoglucuronide and diglucuronide in the bile collected from group A and B. The two glucuronides accounted for more than 95% of the bile pigments, less than 5% being unconjugated bilirubin, This profile is similar to that seen in normal Wistar rats. In groups C, bile pigment analysis following the second infection showed no significant conjugated bilirubin excretion detected in the bile.

This example demonstrates that the level of preexisting antibodies against adenoviruses can be suppressed by oral instillation of the major viral proteins into preimmunized rats. This procedure made it possible to readminister the virus with repeated rounds of transgene expression. The reduction of antibody titers occurs slowly because of the relatively long half-life of immunoglobulins, but importantly, the antibody levels continued to decrease in the tolerized group despite the second injection of Ad-hBUGT₁. The findings indicate that the presence of antibodies in titers of up to 1 :2⁷ does not impede gene transfer using adenoviral vectors. The lack of a detectable metabolic effect after the second administration of the virus in the non-tolerized (BSA-treated) group suggests that the strong secondary humoral or CTL response that resulted from the second injection may he responsible for attenuating the transgene expression by cleaning the recombinant virus or virally infected hepatocytes.

### Example 5. Oral Tolerization to Recombinant Adenovirus is Transferable

Although the preceding examples have demonstrated the induction of tolerance to recombinant Adenoviruses, there may be circumstances where the tolerization cannot be done in the subject. For instances it has previously shown that the presence of inmmunosuppressive drugs such as cyclosporin A may prevented the induction of oral tolerance (Fukushima. A. Whitcup, S.M.. Nussenblatt, R.B., and Gery, I. "In Oral Tolerance; Mechanism and Applicaiions" (1996) 376-378, H.L. Weiner and L.F, Mayer (eds,) The New York Acadamy of Sciences, New York, N.Y., incorporated herein by reference). Presumadly other circumstances that have shut down some of the immune system could also abrogate induction. Under such circumstances it may be useful to induce oral tolerization in one subject (donor) and then transfer this tolerance to a second subject (recipient).

*Adoptive transfer of tolerance:* To determine whether the intestinal Wall or splenic lymphocytes from the tolerized rats are capable of producing tolerance upon transplantation into naive rats, donor rats from groups A and C1 12 rats from each group) from Examples 1 and 2 were killed at the end of the experiment and single suspensions of lymphocytes derived from the spleen or the small intestine were prepared as described previously for Cytotoxic T lymphocyte assays. The cells were resuspended in PBS immediately before transplantation. Recipients rats were sublethally irradiated with 600 rad total body irradiation, 24 hr prior to intravenous injection of 5x10⁷ - 1 x10⁸donor cells in 0.5 ml PBS. A total of eight rats were studied, four received the cells from group A donor rats, and four from group C rats (in each group, 2 rats received donor splenocytes, 5x10⁸ cells, and two received donor gut wall lymphocytes, 5x10⁷ cells). All rats were injected with Ad-hBUGT. twice, one day after cell transplantation and 98 days later. Serum bilirubin levels, pigments excreted in bile and anti-adenoviral cellular and humoral immune responses were determined as described before.

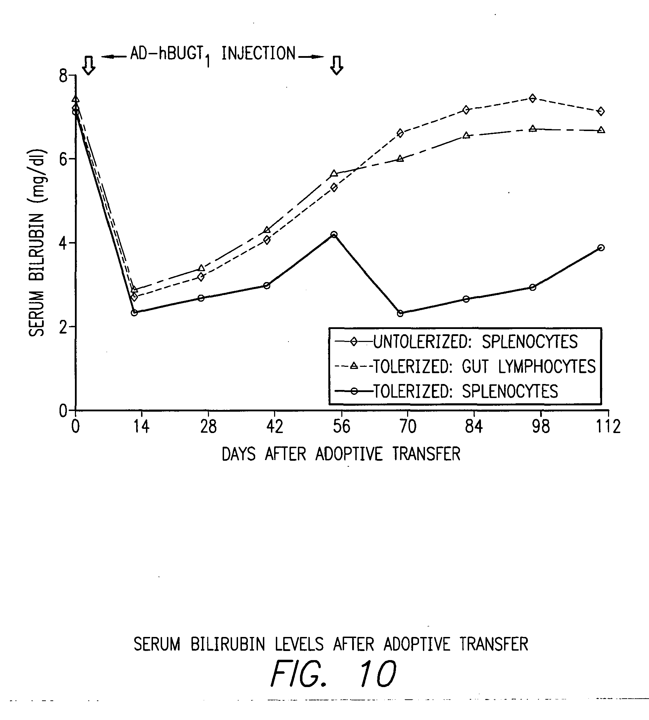

*Evaluation of Adoptive* transfer o*f to*/*arance:* Levels of serum bilirubin in this example are shown in Figure 10. Adoptive transfer of the tolerance was seen only in the two rats receiving the splenocytes from group A. Following AdhBUGT administration, these rats showed a metabolic effect similar to that observed in the tolerized rats from group A described in Example 2. Moreover, one of these rats did not develop anti adenovirus antibodies and the other mounted only a low tiler antibody response. In contrast, when lymphocytes untolerized donors or lymphocytes from the wall of tolerized donors were used, adoptive transfer of the tolerance did not occur anti serum bilirubin returned to levels seen prior to administration of the recombinant adanovirus. Upon a second injection of the virus, these rats failed to show matabolic evidence of BUG1,expressionin the recipients of cells from group C rats, all the rats developed marked anti-adenoviral humoral and recipients of cells from group C rats, all the rats developed a marked anti-adenoviral humoral and cellular immune response, and all lost the transgene effect within 6 weeks of the injection

This example demonstrates that the induction of oral tolerance in one subject is a state that can be transferred into a second subject.

### Example 6 Oral tolerizationto,Recombinant Adenovirus in rabbits and demonstration that tolerance is also established to a non-native transgene

In the previous examples, oral tolerization has provided a stable expression of a recombinant Adenovirus and the tolerization was sufficient to enable expression after a readministration of this Adenovirus vector, However, these examples were performed using rats as subjects and the gene being expressed (BUGT) although derived from a human source may be sufficiently similar to the the native product that there may not have been an elicitation of an immune reaction to the transgene The absence of BUGT gene expression after the second administration may have been a reaction to the vector alone. The following example differs from the previous example in that rabbits are used as the subjects and the gene used in the first and second recombinant Adenovirus administrations is /*acZ* which is derived from *E. coli.*

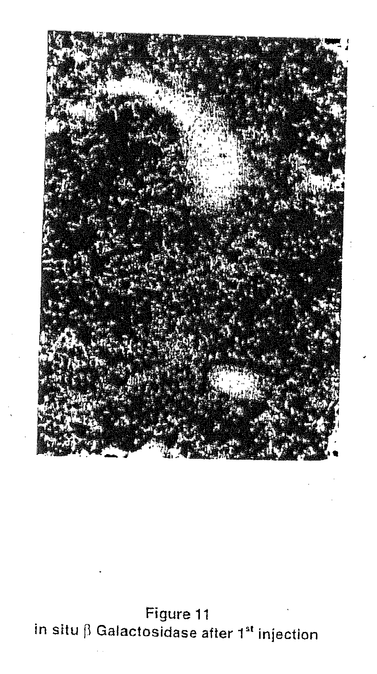

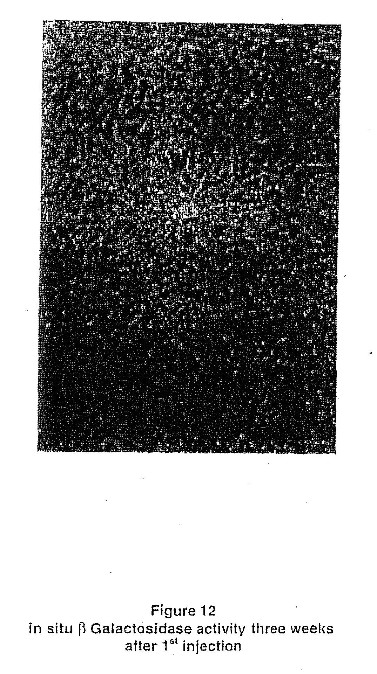

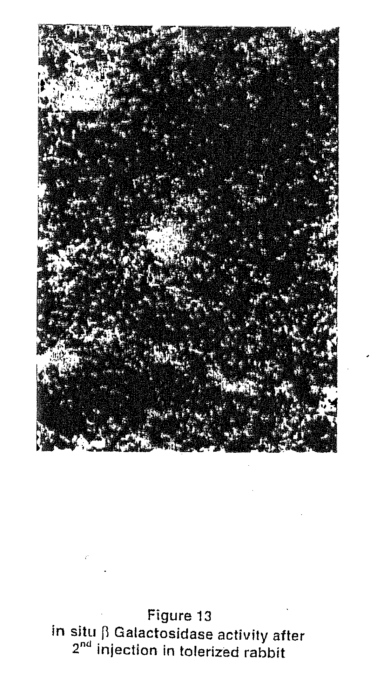

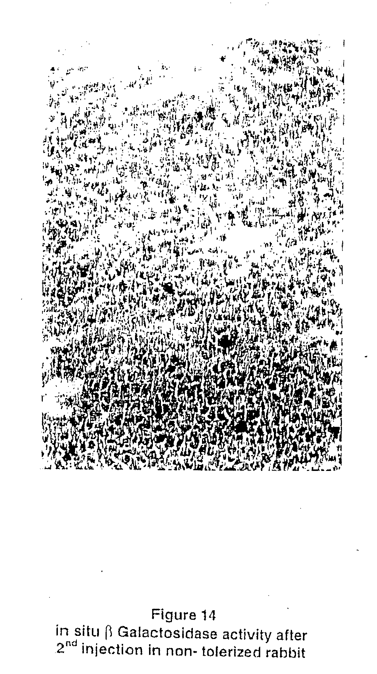

Details of the methods used for induction and evaluation of oral tolerance are as described for the previous examples with the Gunn rats with the following exceptions:
a) the rabbits were fed antigens orally rather than using intubation
b) Due to their larger size, the amount of antigen used for tolerization was increased proportionally (10mg instead of 1 mg) *Evaluation of oral tolerance;* Figure 11 shows that there is a high level of expression of the lacZ gene after introduction into rabbit hepatocytes by adenovirus As seen in the rat system, this is a transient effect and most activity has disappeared after three weeks (Figure 12). When the rabbit is tolerized by oral administration of the Ad.hBUGT₁ vector, a second injection with the recombinant Adenovirus allows efficient expression of the lacz gene (Figure 13). On the other hand, without this tolerization, a second injection is incapable of expressing any significant levels of b-galactosidase activity (Figure 14). This example demonstrates that the present invention is not restricted to rats alone and works efficaciously in other animals. Furthermore, the high level of expression of *lacZ* after the second administrations of recombinant adenovirus demonstrated that there was tolerization to an enzyme that is not native to the subject.

### Examples 7

### Example: Delivery of Protein Molecules to Cells

Bovine Serum Albumin was labeled by conjugation with both biotin and fluorescein (BSA-BF) or with fluourescein alone (BSA-F) by the following method:
BSA-BF: BSA (68 mg) was dissolved in 4.8 ml of 0.2M borate buffer, pH 9.0. Biotin-21-MHS ester (4 umoles) in 2 ml of DMF was added dropwise, and the mixture was incubated at room temperature for 2 hours. Fluorescein isothiocyanate (2 umoles) in 200 ul DMF was added and the mixture was incubated overnight at room temperature. The mixture was evaporated to dryness in a rotary evaporator and then redissolved in 5 ml H₂O and applied to a G-25 column equilibrated with 50 mM Tris buffer, pH 8.0. The fluoresceinated fractions were collected and stored at 0ºC.
BSA-F: This was prepared by the above procedure except that the reaction with biotin 21-NHS was omitted.

U937 cells were grown to approximately 10⁶/ ml and centrifuged, washed in growth medium (RPMI), suspended in 3.5 ml of RPMI , and 0.8 ml was placed in each of four 35 mm wells. BSA-BF and BSA-F, each at a concentration of 20 mg/ml, were added to the four wells together with 200 ul of RPMI as follows:
1) 20 ul BSA-F
2) 50 ui BSA-F
3) 20 ul BSA-BF
4) 50 ul BSA-BF.

The cell suspensions were incubated overnight at 37°C. One ml of cell suspension was placed in 2.0 ml of RPMI and centrifuged at 1000 rpm for 5 minutes. The supernatants were decanted and the cells were resuspended in 200 ul of RPMI. Samples of each cell suspension were placed on a microscope slide and examined using a fluorescent microscope. The following was observed:
1) 20 ul BSA-F: Against a slight background of fluorescense, fewer than 5% of the cells displayed any fluorescence,
2) 50 ul BSA-F: Against a slight background of fluorescense, fewer than 5% of the cells displayed any fluorescense,
3) 20 ul BSA-BF: Approximately 40% of the cells displayed an intense fluorescense,
4) 50 ul BSA-BF:. Approximately 40% of the cells displayed an intense fluorescense.

Evidence that the biotinylated BSA entered cells was provided by observations of cells that were undergoing mitosis as indicated by the presence of two nuclei in a single cell. Such cells displayed a bright fluorescense that was confined to the nuclei.

### Examples 8 TGF-b₁ levels as a marker for oral tolerization

The following example demonstrates the method for establishing that tolerization has been conferred by measuring TGF-b₁ levels The samples were derived from the rats described in Examples 1 and 2.

***Serum TGF-b1*** /*evels:* TGF-b1 levels were measured by a "sandwich" ELISA using Genzyme Diagnostics kit according to manufacturers' instructions. Serum TGF.b 1 levels were measured in three rats from each group after each injection, on days 8 and 101.

### Measurement of TGF-b 1 secreted by intestinal lymphocytes and splenocytes,

For the extraction of gut wall lymphocytes, the small intestines were removed from 2 rats each from the tolerized and the control groups an day 101, and placed in RPMI medium supplemented with 15% FBS. The intestines were cuts into 1 cm segments, flushed with the medium, opened by cutting longitudinally and transferred into fresh medium, rinsed four times with PBS and placed in PBS (calcium and magnesium free), containing 1mM EDTA and 1mM dithiothretol (DTT). Fragments were stirred for 30 minutes at 37°C and the exfoliated cells were harvested by decanting the PBS after tissue fragments had settled. Cells were passed through nylon wool, pelleted by centrifugation for 5 minutes, suspended in 10 ml of 40% Percoll and layered over a cushion of 70% Percoll. Cells were then centrifuged for 20 min at 600 x g and the gut wall lymphocytes at the 70%-40% interface were harvested.

For enrichment of antigen presenting cells, lymphocytes were harvested from the spleens as described above. Cells were then suspended in 4 ml of RPMI containing 5% fetal calf serum (FBS) and 4 ml ol RPMI containing 5% FBS and 14.5 g% metrizamide. After centrifugation at 1800 x g for 20 min, at room temperature, the interface containing an enriched population of macrophages and dendritic cells was collected.

For determination of TGF-b1 secretion, intestinal wall or splenic lymphocytes from untreated Gunn rats, rats from the control groups C and D, and rats from the tolerized group A after each injection, on days 8 and 101 (two rats from each group) were plated on tissue culture dishes 15x10 /10 cm plate) and grown in serum-free media. 1 x10⁸ antigen presenting cells were added per plate along with 50 mg of artenoviral protein extracts as the activating antigen. After 72 hr of culturing, TGF-b1 secreted into the media was quantitied by ELISA as described above.

*Measurements **of** Serum TGF_{b1} concentrations and TGFb₂ secretion in vitro:* Serum TGFb₁ levels were increased to >170 ng/ml after each injections of the recombinant adenovirus in rats that were given the 1 mg/day dose of adenoviral proteins before the injection of recombinant viruses (group Al. in rats that received BSA or no protein before the virus injection (Groups C and D) serum TGF levels were 30-35 ng/ml (p<0.005) The levels in normal untreated Gunn rats are 18-26 ng/ml.

*In vitro* assays for evaluation of TGFb₁ levels after exposure of splenocytes and gut wall lymphocytes to adenoviral antigens were done in two rats from each group after each virus injection. Low levels (>4 ng/ml) of TGFb₁were present in the supernatant of splenocytes and gut wall lymphocytes cultures from the rats that were administered BSA or no protein (group C and D1: similar levels of TGFb₁ were observed using gut wall lymphocytes from tolerized rats (normal levels: 2.1-3,6 ng/ml). ln contrast, splenocytes from rats tolerized by feeding adenoviral proteins (group A) secreted significantly greater amounts of TGFb₁ [24-26 ng/ml) after exposure to viral proteins.

### Example 9 Assessment of various cytokines as markers for oral tolerization

The following example demonstrates the method for establishing that tolerization has been conferred by measuring the levels of various cytokines The samples were derived from the rats described in Examples 1 and 2

*RT-PCR for rat cytokine mRNAs:* These assays were performed on day 101 on two rats from each of groups A, C, D and E. After culturing the lymphocytes with the viral antigens and antigen presenting cells as described above, cells were harvested and mRNA levels for rat IL-2, 4, 6, 10, IFN-g and TGF-b1 were determined by reverse transcription-primed polymerase chain reaction (RT-PCR One mg of RNA was used as template for each sample. Amplimers for rat glyceraldehyde-3-phosphate dehydrogenase (GPDH) were used as an internal Control for the RT-PCR.

*Results of RT-PCR for rat cytokines:* RT -PCR.was performed on days 8 and 101 on RNA extracted from and gut wall lymphocyte cell cultures from the various groups. Positive bands for IL-2, 4, 10, and TGFb1 were found in splenocytes from rats tolerized by adenoviral protein administration (group Al, but not from rats that had received BSA or no protein at all (groups C or D). Gut wall lymphocytes cell cultures from the tolerized rats (groups A), as well as from rats that received BSA or no proteins (groups C or D), were negative for these cytokine mRNAs. In contrast, IFNg was negative by RT-PCR in splenocytes from the tolerized rats in group A, but was found in spienocytes from rats from control groups C and D. Gut wall lymphocytes (GW) showed similar results to non-tolerized rats. 1L6 was detected in all tested groups and probably behaves as a non specific acute phase reactant

### Examples 10. Oral Tolerization for Treatment of Colitis

In this example rats that had undergone oral tolerance to proteins extracted from the colons of colitis-afflicted rats demonstrated reduced pathogenesis. Tolerizing treatment was administered simultaneously with the induction of colitis.

**Methods.** Colitis was induced by intracolonic installation of trinitrobenzenesulfonic acid (TNBS). Treated rats received 5 oral dosages of proteins extracted from diseased colons every other day starting on the day of colitis induction. Control rats received similar doses of bovine serum albumin.

**Results.** Control non-tolerized rats developed severe colitis manifested by severe diarrhea and macroscopically and microscopically severe bowel disease. Feeding of colon proteins enabled induction of anti-coionimmune hyporesponsiveness manifested by: decreased diarrhea with marked decrease in the degree of colonic ulceration, intestinal and peritoneal adhesions, wall thickness and edema. There was marked reduction in the percentage of the injured colonic area out of the total colonic wall and a reduction in colon weight. Microscopic parameters were also improved in tolerized animals which showed a marked reduction in inflammatory response and mucosal ulceration.

The role of suppressor lymphocytes in tolerization was evaluated by testing whether lymphocytes from tolerized rats can adoptively transfer the tolerance to naive animals. Here, splenocytes were harvested from both groups of rats and transplanted into sublethally irradiated animals. The TNBS-induced colitis was markedly alleviated in recipients of spienocytes from tolerized rats. In contrast, in those animals that received lymphocytes from non-tolerized control rats,'severe disease developed.

### Example 11. Treatment of Graft Versus Host Disease (GVHD) through induction of Oral Tolerance

The ability to induce tolerance in a donor towards recipient tissue can be performed through feeding of recipient lymphocytes.

Methods. In order to induce GVHD donor spleen cells will be injected intravenously into sublethally irradiated BALB/c mice. Donor mice (B10D2 males) will be fed on alternate days for 10 days with protein extracted from recipient lymphocytes. The cells will be prepared by isolating peripheral blood lymphocytes from recipient mice (Bafb/c females). Quantitative assessment of the proteins in the cells will be made using standard methods.

**Procedures.** Ten donor mice will be fed with 5 doses of the antigens. Ten control mice will be fed with BSA. On day 12, two days after completion of the feeding, spleens will be harvested and will be transplanted in the recipient mice. The mice will be monitored for the following parameters:
a) determination of tolerance induction by mixed lymphocyte reaction assays;
b) induction of GVHD by skin biopsies and skin collagen determination; and
c) induction of liver and small bowel GVHD.

Many obvious variations will be suggested to those of ordinary skill in the art in light of the above detailed description and example of the present invention. All such variations are fully embraced by the scope and spirit of the Invention as defined by the claims that follow.

**Furthermore, the present Invention relates to the following items:**
1. A process for producing selective immune down regulation in a subject to an infectious bacterial agent comprising introducing to said subject a reagent or a combination of reagents capable of producing selective immune down regulation and comprising a component or components or fragments thereof of said infectious agent.
2. The process of item 1, wherein said infectious bacterial agent comprises streptococcus.
3. The process of item 2, wherein said streptococcus is the causative agent for rheumatic fever or glomerular nephritis.
4. A transplantation process comprising the steps of:
   establishing selective immune down regulation in a recipient subject to the antigens of a donor comprising a member selected from the group consisting of MHC Class I, MHC Class II and histocompatibility factors, or a combination of any of the foregoing: and
   introducing into said recipient subject cells, tissue, or organs, or components thereof from said donor.
5. A transplantation process comprising the steps of:
   establishing selective immune down regulation in a donor to an antigen or antigens of a recipient, said establishment being carried out by introducing to said donor cells, tissue, organs, or components thereof from said recipient, hereby establishing selective immune down regulation in said donor to said recipient antigen or antigens prior to transplanting into said donor the cells, tissue, organs, or components thereof; and
   transplanting thereafter into said recipient the cells, tissue, organs, or components thereof from said donor having selective down immune regulation.
6. The transplantation process of item 5, wherein said recipient antigen or antigens are selected from the group consisting of immune cells, bone marrow cells and T cells, or a combination of any of the foregoing.
7. The transplantation process of item 5, wherein said recipient cells, tissue or organs, or components are selected from the group consisting of immune cells or bone marrow cells or T cells.
8. The transplantation process of item 5, wherein said donor antigen comprises a member selected from the group consisting of MHC Class I, MHC Class 11, and histocompatibility factors, or a combination of any of the foregoing.
9. A process for treating Crohn's disease in a subject, comprising the steps of establishing selective immune down regulation in said subject to inflamed intestine by introducing into said subject components, cells, tissues or organs derived from allogeneic sources, xenogeneic sources and autologous sources, and immunologically equivalents, or combinations thereof.
10. A method of treating diabetes in a subject by establishing in said subject selective immune down regulation to non-insulin epitopes comprising a member selected from the group consisting of pancreatic cells, insulin receptors, fatty cells and liver cells, or component thereof, said epitopes being derived a source selected from allogeneic sources, xenogeneic sources and autologous sources, and immunologically equivalents, or combinations thereof.

## Claims

1. Use of antigens of a donor comprising a member selected from the group consisting of MHC Class I, MHC Class II and histocompatibility factors, or a combination of any of the foregoing for the preparation of a pharmaceutical composition for establishing selective immune down regulation in a recipient prior to introducing into said recipient subject cells, tissue, or organs, or components thereof from said donor.

2. Use of cells, tissue, organs, or components thereof from a recipient for the preparation of a pharmaceutical composition for establishing selective immune down regulation in a donor to an antigen or antigens of the recipient, thereby establishing selective immune down regulation in said donor to said recipient antigen or antigens prior to transplanting into said donor the cells, tissue, organs, or components thereof, whereby cells, tissue, organs, or components thereof from said donor having selective down immune regulation are then to be transplanted into said recipient.

3. The use of claim 2, wherein said recipient antigen or antigens are selected from the group consisting of immune cells, bone marrow cells and T cells, or a combination of any of the foregoing.

4. The use of claim 2, wherein said recipient cells, tissue or organs, or components are selected from the group consisting of immune cells or bone marrow cells or T cells.

5. The use of claim 2, wherein said donor antigen comprises a member selected from the group consisting of MHC Class I, MHC Class II, and histocompatibility factors, or a combination of any of the foregoing.

6. Use of a derivative of a MHC protein or analogue to treat immune related disorders.


SEQUENCE LISTING

<110> ENZO THERAPEUTICS, INC.

Roy-Chowdhury et al., Jayanta

<120> Novel Processes Implementing Selective Immune Down Regulation (SIDR)

<130> Enz-55 (U.S.)

<140> 08/808,629

<141> 1997-02-28

<160> 2

<170> PatentIn version 3.0

<210> 1

<211> 21

<212> DNA

<213> Artificial Sequence

<400> 1
aaggaaaggg tccgtacagc a 21

<210> 2

<211> 19

<212> DNA

<213> Description of Artificial Sequence : Antisense primer

<400> 2
ccagcagctg cagcagagg 19